# EUROPEAN PATENT APPLICATION

(11) **EP 2 993 171 A1**
(43) Date of publication of application: **09.03.2016**
(21) Application number: 14183635.3
(22) Date of filing: 04.09.2014
(51) Int. Cl.: C07D 213/81, C07D 213/82, C07C 275/16, C07D 231/14, C07D 241/24, C07C 231/02, A61K 31/44, A61P 35/00

(54) **Method for the production of 18F-labeled PSMA-specific PET-tracers**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

Novel efficient, time-saving and reliable radiofluorination procedures for the production of ¹⁸F-labelled active esters via nucleophilic substitution of the corresponding onium precursors with ¹⁸F⁻ are described. The active ester including [¹⁸F]F-Py-TFP and [¹⁸F]TFB produced by one of these methods was used to prepare PSMA-specific PET tracers such as [¹⁸F]DCFPyL. The key advantages of these inventive methods are efficiency, short time of preparation and excellent amenability to automation. A pharmaceutical composition containing at least one PSMA-specific PET tracers prepared by the inventive method is useful for positron emission tomography (PET) imaging, especially imaging prostate tumor.

## Description

Novel efficient, time-saving and reliable radiofluorination procedures for the production of ¹⁸F-labeled active esters via nucleophilic substitution of the corresponding onium precursors with ¹⁸F⁻ are described. The active ester including [¹⁸F]F-Py-TFP **3c** and [¹⁸F]TFB **3a** produced by one of these methods was used to prepare PSMA-specific PET tracers such as [¹⁸F]DCFPyL **1-10.** The key advantages of these inventive methods are efficiency, short time of preparation and excellent amenability to automation. A pharmaceutical composition containing at least one PSMA-specific PET tracers prepared by the inventive method is useful for positron emission tomography (PET) imaging, especially imaging prostate tumor.

### Background of the invention

Amongst imaging technologies PET (**P**ositron **E**mission **T**omography) plays a very important role due to its outstanding potential to visualize physiological processes at the molecular level in real time. PET is therefore essential in clinical diagnostics and has gained major significance in drug development. Beside technical improvements, PET benefits mainly from innovations in the field of tracer development, comprising both progress in labelling strategies and an intelligent design of selective molecular probes with the capability to visualize molecular targets involved in physiological and pathophysiological processes. A prerequisite for the latter is a deepened insight into the biology underlying normal or diseased states at the molecular level. Molecular probes for PET-imaging must be labeled with suitable β⁺-emitting nuclides. Among the spectrum of easy available radionuclides ¹⁸F-fluorine is still the nuclide with the highest impact in PET research. This is mainly based on the excellent nuclear properties of ¹⁸F in comparison to other cyclotron-produced nuclides. Decay characteristics of ¹⁸F [E(β⁺) = 630 keV, abundance: 97%; t_{½} = 109.8 min] make it an ideal PET-isotope with respect to half-life and resolution.

Numerous methods for ¹⁸F labelling have been developed to prepare tailor-made probes which allow visualizing biochemical processes of interest. The vast majority of ¹⁸F-labelling techniques are based on aliphatic and aromatic nucleophilic substitution reaction with ¹⁸F⁻. Sometimes, ¹⁸F-labeled small molecules can be obtained in one step from the proper labelling precursor. However, protecting groups are often required for functionalities in the molecule which may interfere with the radiofluorination reaction. Relatively harsh reaction conditions for radiofluorination are normally incompatible with sensitive molecules including proteins and the majority of peptides. In this case indirect radiofluorination via ¹⁸F-labeled prosthetic groups is the only alternative. Radiofluorinated active esters - amine-reactive prosthetic groups - are among the most widely used radiolabeled building blocks.

In all previously described radiosyntheses of ¹⁸F-labelled active esters [¹⁸F]fluoride should be preliminary taken up in an aqueous or aqueous/organic solution of moderately strong or weak bases to give the corresponding [¹⁸F]fluoride salt. Usually K, Cs or tetraalkylammonium carbonates/bicarbonates are used. In case of K salts aminopolyethers are routinely added to enhance the nucleophilicity of ¹⁸F⁻. In case of Cs and tetraalkylammonium salts enhancement of ¹⁸F⁻ nucleophilicity is achieved as a result of charge separation based on the great difference between the sizes of counter ions. However, active esters are limitedly stable under basic conditions. Consequently, a majority of them including N-succinimidyl 4-[¹⁸F]fluorobenzoate ([¹⁸F]SFB) could not be usually prepared in one step in acceptable RCYs. Only few of them such as 6-[¹⁸F]fluoronicotinic acid 2,3,5,6-tetrafluorophenyl ester ([¹⁸F]F-Py-TFP) could be prepared via direct radiofluorination. Furthermore, water substantially diminishes nucleophilicity of ¹⁸F⁻ due to tenacious hydration. Consequently, water removal using repetitive azeotropic drying with acetonitrile is usually mandatory.

[¹⁸F]SFB is most commonly used for the preparation of radiolabelled biomolecules. However, its broad application is hampered by tedious preparation procedures. Some of them are summarized in Table 1.

**Table 1: Selected methods for the preparation of [¹⁸F]SFB.**

| Steps | Precursor | R | Process | Time [min] | RCY [%] | Ref |
|---|---|---|---|---|---|---|
| 3 | 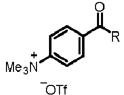 | H | 3-step, 3-pot, 3 separations (SPE & HPLC) | 80 | 30-35 | 1 |
| | | OEt | 3-step, 2-pot, 2 separations (SPE) | 78 | 41-51 | 2 |
| | | | 3-step, 1-pot, 1 separation (SPE) | 60 | 44 | 3 |
| | | O*t*Bu | 3-step, 2-pot, 2 separations (SPE) | 68 | 34-38 | 4 |
| 2 | 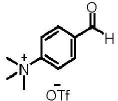 | - | 2-step, 2-pot, 2 separations (SPEs or SPE & HPLC) | 100 (for 2 SPE); 160 (for SPE & HPLC) | 30-40 | 5 |

Ref. 1 - G. Vaidyanathan, M. R. Zalutsky, Nucl. Med. Biol. 1992, 19, 275-281.; Ref. 2 - H.-J. Wester, K. Hamacher, G. Stocklin, Nucl. Med. Biol. 1996, 23, 365-372.; Ref. 3 - S. Guhlke, H. H. Coenen, G. Stöcklin, Appl. Radiat Isot. 1994, 45, 715-725.; Ref. 4 - E. D. Hostetler, W. B. Edwards, C. J. Anderson, M. J. Welch, J. Label. Compd. Radiopharm. 1999, 42, S720-S722.; Ref. 5 - M. Glaser, E. Arstad, S. K. Luthra, E. G. Robins, J. Label Compd. Radiopharm. 2009, 52, 327-330.

All described [¹⁸F]SFB radiosyntheses consist of 2-3 reactions and multiple operation steps. For example, according to Wester et al.( Nucl. Med. Biol. 1996, 23, 365-372) [¹⁸F]SFB was prepared via ethyl 4-[¹⁸F]fluorobenzoate (Scheme 1).

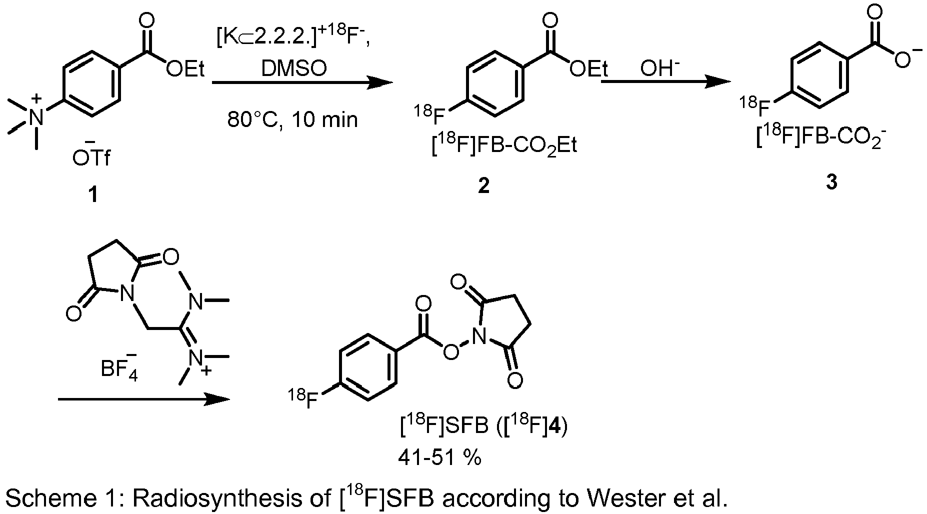

Accordingly, ¹⁸F⁻ target water was added to a solution of K₂CO₃/K₂C₂O₄ and K2.2.2 (Kryptofix 222) in aqueous MeCN. The solvent was removed under reduced pressure in a flow of nitrogen and the residue was two times azeotropically dried by using MeCN. The residue was taken up in a solution of *N,N,N*-trimethyl-4-carbomethoxyanilinium triflate in DMSO. The reaction mixture was heated at 90-110 °C for 6 min. Thereafter, 1 M NaOH was added and the mixture was heated at the same temperature for a further 10 min. Afterwards, it was acidified with 1 M HCl and diluted with water. The intermediate 4-[¹⁸F]fluorobenzoic acid was purified by SPE (solid phase extraction) using polystyrene and cation exchange cartridges. Tetramethylammonium hydroxide was then added to the methanolic solution of the intermediate and MeOH was removed under reduced pressure at 90°C. The residue was two times azeotropically dried by using MeCN. To the residue a solution of TSTA (*N,N,N'N'-*tetramethyl(succinimido)uronium tetrafluoroborate) in MeCN was added and the mixture was heated at 90 °C for 2 min. [¹⁸F]SFB was finally isolated by SPE using a polystyrene resin.

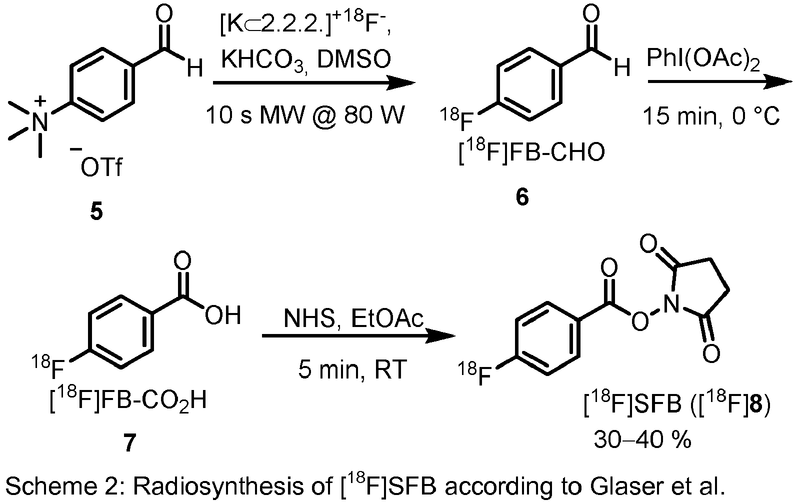

Glaser et al. (J. Label Compd. Radiopharm. 2009, 52, 327-330) produced [¹⁸F]SFB via 4-[¹⁸F]fluorobenzaldehyde ([¹⁸F]FB-CHO) (Scheme 2). To a solution of K₂CO₃ and K2.2.2 in aqueous MeCN irradiated target water was added. The solvent was removed under reduced pressure in a flow of nitrogen and the residue was three times azeotropically dried by using MeCN. The residue was taken up in a solution of *N,N,N*-trimethyl-4-formylanilinium triflate in DMSO or DMF. The reaction mixture was briefly heated using microwave energy. The mixture was transferred on a silica gel cartridge and most of the solvent was removed by flushing with nitrogen. The intermediate [¹⁸F]FB-CHO was fractionally eluted using anhydrous ethyl acetate. One fraction (0.5 mL) was cooled to 0 C. lodobenzene diacetate (BAIB) was added, the mixture was stirred at 0°C for 15 min and allowed to reach ambient temperature for 5 min. It was decanted and the supernatant was purified via SPE or HPLC to afford [¹⁸F]SFB in EtOAc/hexane solution. Before [¹⁸F]SFB can be used for labelling of EtOAc/hexane-insoluble proteins or peptides, it should be taken up in an water-miscible solvent.

Additionally, the high hydrophobicity of the fluorobenzoyl group limits its application for the labelling of small molecules and shorter peptides. Therefore, several alternatives to SFB were proposed. The most interesting one is 6-[¹⁸F]fluoronicotinic acid 2,3,5,6-tetrafluorophenyl ester ([¹⁸F]F-Py-TFP) first published by Olberg et al. (J. Med. Chem. 2010, 53, 1732-1740). [¹⁸F]F-Py-TFP could be prepared using a one-step procedure in moderate radiochemical yield (RCYs) of 40-50% (Scheme 3). Additionally, [¹⁸F]F-Py-TFP is more hydrolytically stable compared to [¹⁸F]SFB. Moreover, it provides more hydrophilic radiolabelled conjugates.

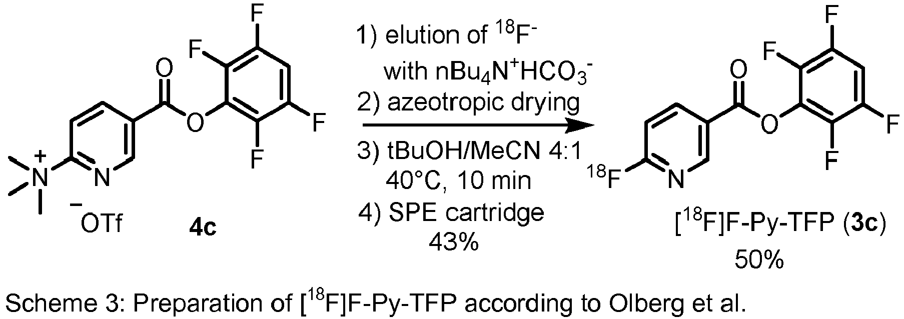

According to Olberg et al. ¹⁸F⁻ was fixed on an anion exchange resin. Thereafter, it was eluted with a solution of tetrabutylammonium bicarbonate in 50% MeCN. The solvent was removed under reduced pressure in a flow of nitrogen and the residue was two times azeotropically dried by using MeCN. After that, a solution of the respective precursor, *N,N,N*-trimethyl-5-[(2,3,5,6-tetrafluorophenoxy)-carbonyl]pyridine-2-aminium trifluoromethanesulfonate 4c, in MeCN/*t*BuOH 2:8 was added to the [¹⁸F]TBAF and the reaction mixture was heated at 40 C for 10 min to give [¹⁸F]F-Py-TFP 3c in 50% RCY. [¹⁸F]F-Py-TFP was purified by SPE on a mixed mode reversed phase cation exchange resin. This synthetic method includes azeotropic drying steps. These preparation steps cause longer synthesis time and this results in lower RCYs of [¹⁸F]F-Py-TFP **3c .** Furthermore, formation of [¹⁸F]F-Py-TFP is accompanied by the concurrent formation of significant amounts of 2,3,5,6-tetrafluorophenyl 6-(2,3,5,6-tetrafluorophenoxy)nicotinate which should be completely separated from the radiolabelled active ester best by HPLC.

In recent years imaging of prostate carcinoma (PCa) with PET isotope labelled PSMA ligands has become of considerable importance in clinical diagnosis. This can be mainly attributed to the high expression of the extracellular localized prostate specific membrane antigen (PSMA) in PCa. Ligands bearing the syL-C(O)-Glu- binding motif exhibit high binding affinity to PSMA. Pomper et al. (WO2010/01493; Clin. Cancer Res. 2011, 17, 7645-7653) exploited this lead structure to prepare 2-(3-{1-carboxy-5-[(6-[¹⁸F]fluoro-pyridine-3-carbonyl)-amino]-pentyl}-ureido)-pentanedioic acid ([¹⁸F]DCFPyL) (Scheme 4). This PET tracer provided a clear delineation of PSMA positive prostate tumor xenografts in mice with an excellent tumor to background ratio.

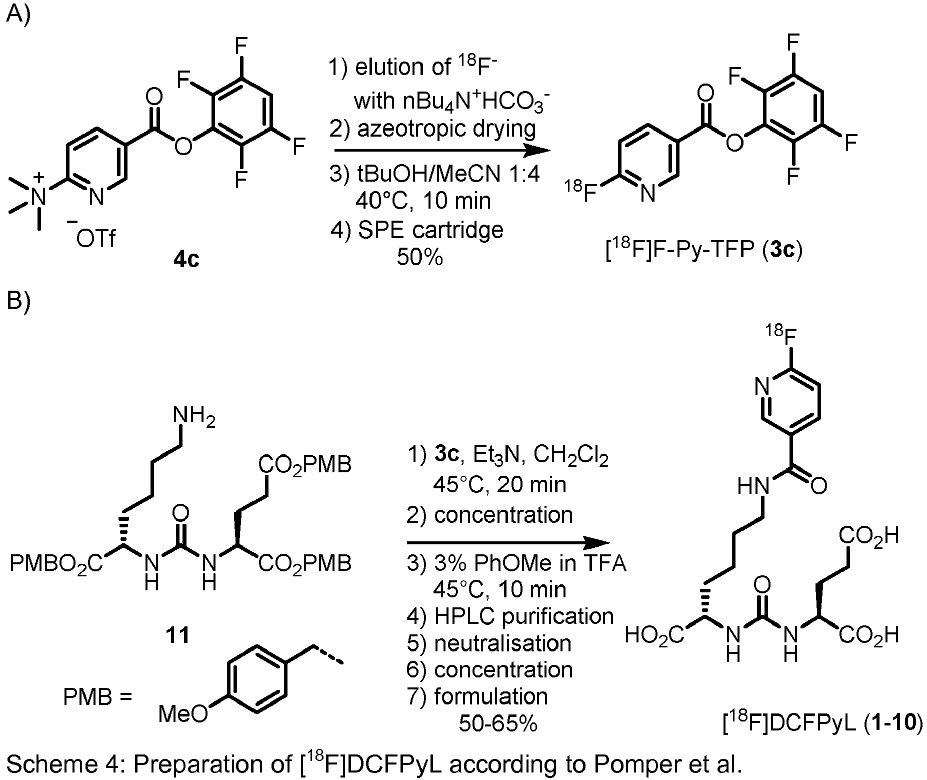

Pomper et al. prepared [¹⁸F]DCFPyL in three steps. First, [¹⁸F]F-Py-TFP was synthesized as reported by Olberg et al. (J. Med. Chem. 2010, 53, 1732-1740). [¹⁸F]F-Py-TFP was eluted from the resin with dichloromethane into a vial containing 1,5-bis(4-methoxyphenyl)methyl 2-[({6-amino-1-[(4-methoxyphenyl)methoxy]-1-oxohexan-2-yl}carbamoyl)amino]pentanedioate [H-DUPA(OPMB)₃] and triethylamine **11** (Scheme 4). The reaction mixture was heated at 45°C for 20 min. Thereafter the solvent was removed with a stream of nitrogen. Anisole in TFA was added, the reaction mixture was heated at 45°C for further 10 min and the desired product was isolated via HPLC using 10% MeCN (0.1% TFA). The fraction containing [¹⁸F]DCFPyL was neutralized with sodium bicarbonate, concentrated to dryness under reduced pressure and reconstituted in PBS to give [¹⁸F]DCFPyL in RCYs of 50-65%. The disadvantages of the above-mentioned method are:
- Application of dichloromethane as a solvent
- Two evaporation steps
- PMB deprotection step using toxic TFA (Scheme 4B step 3)
- Neutralisation step
- Formulation step

It is the objective of the present invention to provide an inventive method for preparation of PSMA selective PET tracer for prostate tumor imaging comprising a simplified procedure for the fast and high yielding preparation of ¹⁸F-labelled active esters and a pharmaceutical composition containing at least one compound (I) prepared by the inventive method for use in positron emission tomography (PET) imaging, especially imaging prostate tumor.

The objective of the present invention is solved by the teaching of the independent claims. Further advantageous features, aspects and details of the invention are evident from the dependent claims, the description, the figures, and the examples of the present application.

### Description of the invention

Accordingly the present invention relates to a method for preparing a compound of the general formula (I): 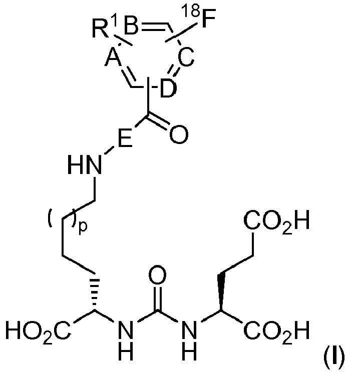 wherein
A, B, C, and D represent independently of each other C-H, C-F or N; and not more than two of **A, B, C,** and **D** represent N;
E represents a covalent bond or 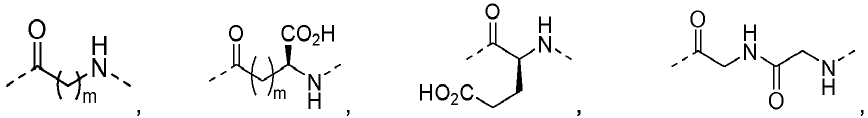 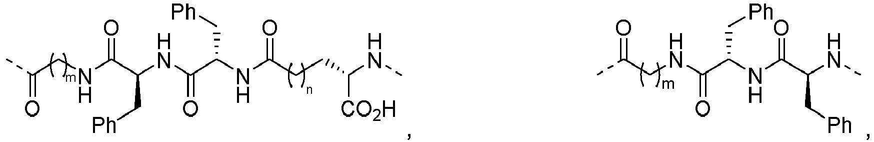 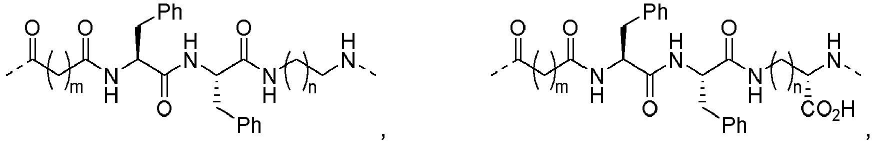 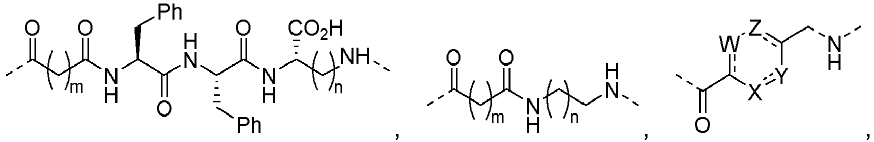 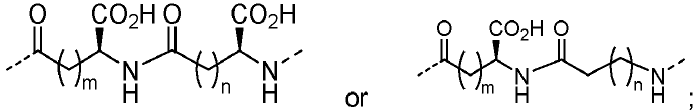
**R¹** represents C₁-C₃ alkyl, C₁-C₃ alkoxy, C₁-C₃ haloalkyl, C₁-C₃ haloalkoxy, C₂-C₄ alkylcarbonyl, C₂-C₄ alkyloxycarbonyl, C₂-C₄ alkylcarboxy, aryloxy, arylcarboxy, cyano, or nitro;
n is an integer selected from 0 to 10;
m is an integer selected from 1 to 18;
**p** is an integer selected from 0 to 10, preferably 0 to 5;
**X, Y, W,** and **Z** represent independently of each other -CH₂-, -CH-, -NH-or -N-;
-̅-̅-̅-̅ represents a single or double bond;
and diastereomers, entantiomers, hydrates, and salts thereof.

This method for preparing the compounds of the general formula (I) comprises the following steps:
(A) providing a solution of a compound of formula (II) in a polar protic solvent or in a solvent mixture containing a polar protic solvent containing at least one base 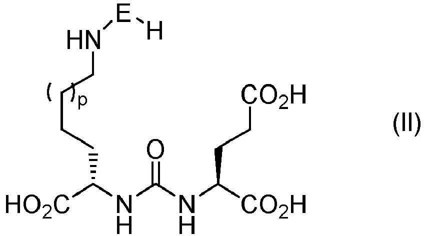 wherein
   E represents a covalent bond or 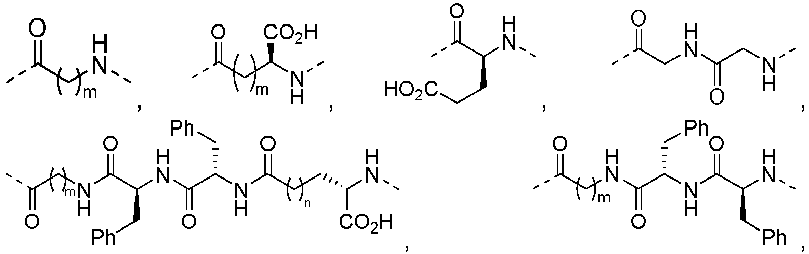 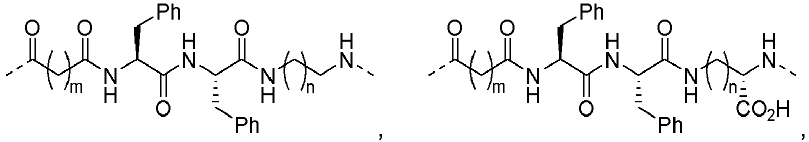 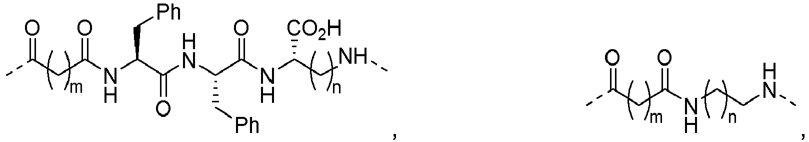 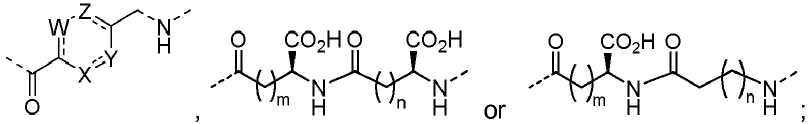
   **R¹** represents C₁-C₃ alkyl, C₁-C₃ alkoxy, C₁-C₃ haloalkyl, C₁-C₃ haloalkoxy, C₂-C₄ alkylcarbonyl, C₂-C₄ alkyloxycarbonyl, C₂-C₄ alkylcarboxy, aryloxy, arylcarboxy, cyano, or nitro;
   n is an integer selected from 0 to 10;
   m is an integer selected from 1 to 18;
   **p** is an integer selected from 0 to 10, preferably 0 to 5;
   **X, Y, W,** and **Z** represent independently of each other -CH₂-, -CH-, -NH- or -N-;
   -̅-̅-̅-̅ represents a single or double bond;
(B) providing a solution of a compound of formula (III) 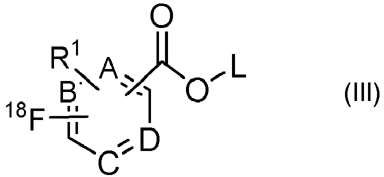 wherein
   **A, B, C, D** and **R¹** have the meanings as defined above, and **OL** represents a leaving group
   in a polar protic solvent or in a solvent mixture containing a polar protic solvent;
(C) mixing the solution of the compound of formula (II) and the solution of the compound of formula (III) and allowing the compound of formula (II) to react with the compound of formula (III) in order to obtain the compound of formula (I);
(D) purifying the compound of formula (I) preferably by using saline (which is isotonic sodium chloride solution).

The residue L of the leaving group OL represents preferably: 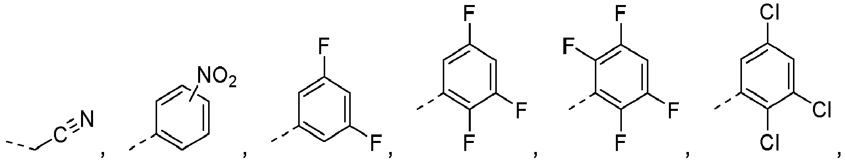 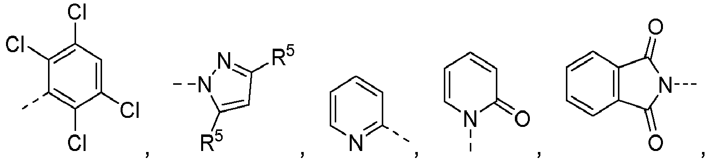 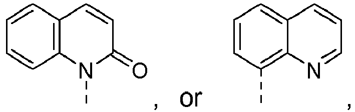 and wherein R⁵ is selected from methyl, ethyl, or n-propyl.

It is also preferred if **A** or **B** or **C** or **D** is nitrogen and the other substituents are CH or CF. More preferred are compounds wherein **A** and **B** are nitrogen and **C** and **D** are CH or CF or wherein **B and C** are nitrogen and **A** and **D** are CH or CF or wherein **C** and **D** are nitrogen and **A** and **B** are CH or CF or wherein **A** and **D** are nitrogen and **B** and **C** are CH or CF or wherein **A** and **C** are nitrogen and **B** and **D are** CH or CF or wherein **B** and **D** are nitrogen and **A** and **C** are CH or CF or wherein **A, B, C, D** are CH or wherein **A, B, C, D** are CF.

Further preferred are compounds wherein the moiety 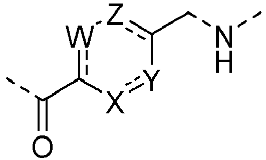 contains an aromatic nitrogen hereterocyclic ring wherein one of **X, Y, W,** and **Z** is nitrogen and the other substituents are CH, or wherein **X** and **Y** or **W and Z** or **X** and **W** or **Z** and **Y** or **X** and **Z** or **W** and **Y** are nitrogen and the other two substituents are CH.

p is preferably selected from 0, 1, 2, 3, 4 and 5, more preferably from 0, 1, 2, 3 and 4, still more preferably from 0, 1, 2 and 3, still more preferably from 0, 1 and 2, and most preferably from 0 and 1.

An embodiment of the present invention refers to any one of the above-described methods, wherein the base of step (A) is an organic nitrogen-containing base or a bicarbonate. The organic nitrogen-containing base is preferably a monoalkylamine, dialkylamine, trialkylamine or an ammonium base, especially a tetraalkylammonium base and more preferably a trialkylamine or tetraalkylammonium base, wherein the term "trialkylamine" and the term "tetraalkylammonium" covers also cyclic amines and cyclic ammonium ions such as N-ethylmorpholine and N-methylpiperidine. Said organic nitrogen-containing base or said bicarbonate is preferably selected from the group comprising or consisting of: Me₄NHCO₃, Et₄NHCO₃, *n*-Pr₄NHCO₃, *i*-Pr₄NHCO₃, *n*-Bu₄NHCO₃, *i*-Bu₄NHCO₃, Et₃N, pyridine, lutidine, collidine, diisopropylethylamine, *n*-Pr₃N, *i*-Pr₃N, *n*-Bu₃N, *i*-Bu₃N, Oct₃N, N-methyl-morpholine, N-ethylmorpholine, N-methylpiperidine, N-ethylpiperidine, *N,N*-dicyclohexylmethylamine, *N,N-*dimethylcyclohexylamine, *N*-methyldibutylamine, *N*-ethyldicyclohexylamine, *N,N-*dimethylbutylamine, and *N,N*-dimethylhexylamine. Said bicarbonate is preferably selected from the group comprising or consisting of: LiHCO₃, NaHCO₃, KHCO₃, CsHCO₃, Me₄NHCO₃, Et₄NHCO₃, *n*-Pr₄NHCO₃, and *n*-Bu₄NHCO₃; and preferably from Me₄NHCO₃, Et₄NHCO₃, *n*-Pr₄NHCO₃, and *n*-Bu₄NHCO₃.

In one embodiment of the present invention, said method comprising the step (C), wherein step (C) is performed at a reaction temperature T2 which is in the range of 20°C to 130°C, preferred 20°C to 100°C, more preferred 30°C to 80°C, most preferred 30°C - 60 °C and during a reaction time t2 which is in the range of 0.1-40 min, preferred 0.1 - 30 min, more preferred 0.5 - 15 min, most preferred 1 - 10 min and at a pH value of the reaction solution which is in the range of 7.0 to 11.0.

More preferably step (C) is performed during a reaction time t2 of 1 to 5 minutes at a pH value of 7.0 to 11.0. Still more preferably step (C) is performed during a reaction time t2 of 1 to 5 minutes at a pH value of 7.0 to 11.0 and at a reaction temperature T2 in the range of 20°C to 80°C and more preferably in the range of 30°C to 60°C.

In the step (D), the compound of the formula (I) is preferred purifed by preparative HPLC. As eluent saline may be suitable for purifying the compound of the formula (I). Said saline is preferred isotonic sodium chloride solution containing 0.90 % (weight/volume, 9g/1000mL) of sodium chloride. Optionally, said saline may further contains5 - 15 % (volume/volume) of EtOH.

Alternatively, phosphate buffered solution can be used for purifying the compound of the formula (I). The phosphate buffered solution usually comprises sodiumphosphate, potassium phosphate and/or phosphoric acid. Optionally said phosphate buffered solution further comprises sodium chloride and/or potassium chloride. Preferred, the osmolarity and ion concentration of said phosphate buffered solution match those of the human body (isotonic). The pH value of said phosphate buffered solution is in the range of 2.0 to 9.0, preffered 4.0 to 8.0, more preferred 6.0 to 8.0 and most preffered 7.0 to 7.8. For example, PBS (phosphate buffered saline) can be used for purifying the compound of the formula (I).

This purification step is a technical advantage compared with the prior art. In the prior art as shown in schem 4, the HPLC purification was performed by using an aqueous elutent containinng toxic acetonitrile and trifluoroacetic acid (TFA). Thus, the solution of the purified compound is not suitable for direct use for PET imagaing. The toxic solvents should be firstly removed and the compound residue should be redissolved in the appropriate buffer solution. In constrast, in according to the present invention the resulting isotonic solution of the purified compound of the formula (I) after the step (D) is ready for application.

Reverse phase column having a good hydrophobic retention and selectivity may be suitable for the purification (e.g. Synergi 4µm Hydro-RP 80A 100×21,2 mm). The phase should be good suitable for the separation of high polar hydrophilic compounds. Retention time should preferentially be less than 10 min by flow rate which is less than 10 mL/min.

In one embodiment of the present invention, any one of the above-mentioned methods further comprises the following step (E) after the step (D):
(E) sterilizing the solution of the compound of formula (I) via sterile filtration.

In one embodiment of the present invention, any one of the above-mentioned methods further comprises the following steps (A1) - (A8) after step (A) and before step (B):
(A1) providing a solution of a compound of formula (IV) in at least one polar protic solvent or in a solvent mixture containing a polar protic solvent, wherein optionally the solution further contains a salt; 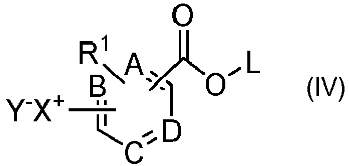 wherein
   A, B, C, D, L (or OL) and **R¹** have the same meanings as defined above;
   X represents **NR²₃, IR³, SR³₂;**
   **Y** represents Br, I, BF₄, O₂CCF₃, OSO₂CF₃, ClO₄, NO₂, OSO₂C₆H₄CH₃, OSO₂CH₃;
   **R²** represents C₁ - C₄ alkyl; and
   **R³** represents aryl;
(A2) providing an aqueous solution of [¹⁸F]fluoride (irradiated [¹⁸O]water);
(A3) loading an aqueous solution of [¹⁸F]fluoride onto an anion exchange resin;
(A4) washing the anion exchange resin with a polar protic solvent or with a polar aprotic solvent;
(A5) flushing of the solvent with air or inert gas flow;
(A6) Eluting of [¹⁸F]fluoride with the solution of the compound of the formula (IV) provided in the step (A1) and diluting of the resulting solution with an aprotic solvent or with at least one C₃ - C₆ alcohol or with a solvent mixture of at least one C₃ - C₆ alcohol and an aprotic solvent; or eluting of [¹⁸F]fluoride with the solution of the compound of the formula (IV) provided in the step (A1), concentrating of the resulting solution and redissolving of the residue in an aprotic solvent;
(A7) allowing the compound of formula (IV) to react with [¹⁸F]fluoride in order to obtain the compound of the formula (III); 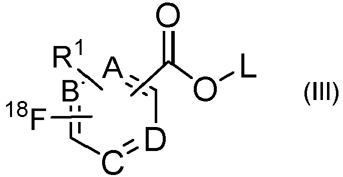 wherein **A, B, C, D, L** (or OL) and **R¹** have the same meanings as defined above; and
(A8) purifying of the compound of the formula (III).

An embodiment of the present invention refers to the above-described inventive method, wherein said polar protic solvent is an anhydrous polar protic solvent, preferably an anhydrous C₁-C₄ alcohol, diols such as 1,4-butanediol or glycols such as diglycol, triglycol or tetraglycol, and especially anhydrous MeOH or anhydrous EtOH or a mixture thereof.

The use of an anhydrous protic solvent is advantageous in the steps (A) - (C) of the inventive method, because the presence of water prevents or impedes coupling reaction between the compound of the formula (II) and the compound of the formula (III). In the presence of water, the active esters of formula (III) having a leaving group -O-L may react with water and form an undesired product i.e. the corresponding carboxylic acid. This results in lower yield of the compound of the formula (I).

For the same reason, in the steps (A1) - (A7), said polar protic solvent is preferably an anhydrous polar protic solvent, more preferred an anhydrous C₁-C₄ alcohol, diols such as 1,4-butanediol or glycols such as diglycol (HO-C₂H₄-O-C₂H₄-OH), triglycol (HO-C₂H₄-O-C₂H₄-O-C₂H₄-OH) or tetraglycol (HO-C₂H₄-O-C₂H₄-O-C₂H₄-O-C₂H₄-OH), and especially anhydrous MeOH or anhydrous EtOH or a mixture thereof. In the steps (A1) - (A7), the presence of water prevents or impedes a nucleophilic aromatic substitution reaction of ¹⁸F⁻ and results in a poor yield of the compound of formula (III).

Said salt in step (A1) is preferably a bicarbonate salt and more preferred selected from the group consisting of Me₄NHCO₃, Et₄NHCO₃, *n*-Pr₄NHCO₃, *i*-Pr₄NHCO₃, *n*-Bu₄NHCO₃ and *i*-Bu₄NHCO₃.

In the steps (A3) and (A4), said anion exchange resin is preferred a silica-based hydrophilic resin or polystyrene-based copolymer containing carbonate or bicarbonate as counter anion and contained in a catridge For example, commercially available Sep-Pak Accell Plus QMA carbonate plus light cartridges from Waters GmbH (Eschborn, Germany) and ChromafixR 30-PS-HCO₃.cartridges from Macherey-Nagel (Duren, Germany) can be used.

In the steps (A4) and (A6), said polar aprotic solvent or said aprotic solvent is preferred MeCN or DMSO and said C₃-C₆ alcohol is preferred t-BuOH.

In the step (A7), the compound of the formula (IV) reacts with [¹⁸F]fluoride in a reaction time in the range of 1 - 20 min, preferred 1 - 15 min, more preferred 5 - 10 min at the reaction temperature in the range of 20 °C to 140°C, preferred 30 °C-130 °C, more preferred 40 °C - 130°C. After the reaction is finished, the reaction mixture is preferred cooled to a temperature in the range of 15 C - 30°C by air cooling.

In the step (A8), purifying of the compound of formula (III) is performed via HPLC or solid phase extraction (SPE). HPLC purification can be carried out using, for example, polymer-based RP columns such as Chromolith SpeedRod or RP C18 or C8 columns like Luna-2 or Nucleosil. SPE purification can be carried out using, for example, Strata X or Sep-Pak C18 cartrigdes.

In one embodiment of the present invention, any one of the above-described methods further comprises the following step (F) after the step (D) or (E):
(F) preparing a pharmaceutical compostion containing the solution of the compound of formula (I).

The compound of the formula (II) may have a good affinity to prostate-specific membrane antigen (PSMA).

Preferred, a compound of the formula (II) selected from the compounds **2a** - **2p:**

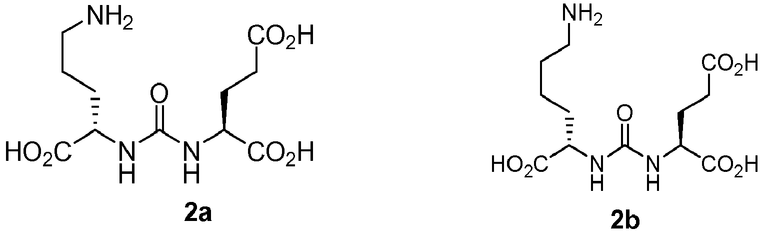

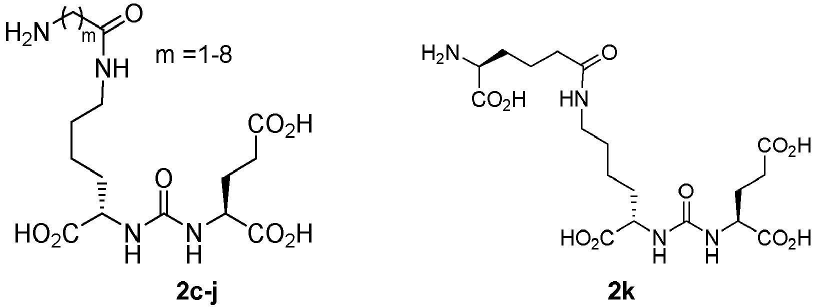

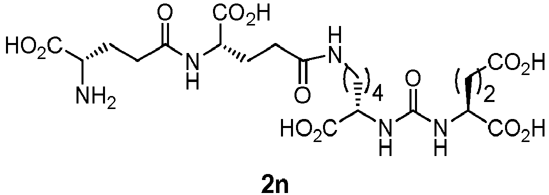

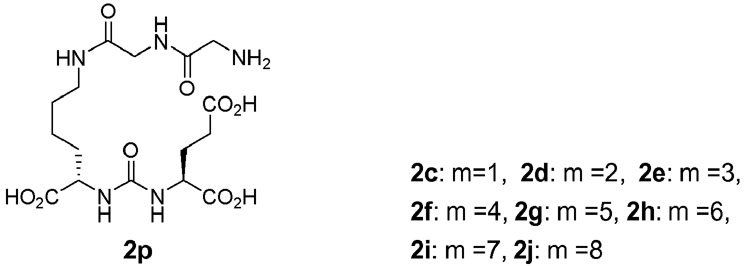

Preferred, a compound of the formula (IV) having any of the subformulae (IV-1) - (IV-5) is used in the above-mentioned step (A1) of the inventive method: 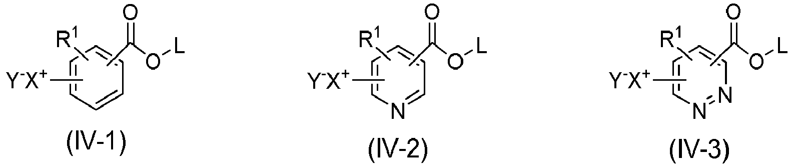 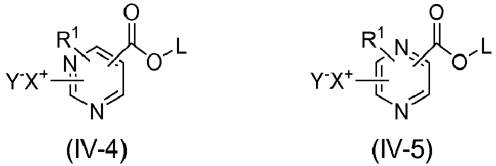 wherein L, R¹, X and Y have the same meanings as defined above.

Also preferred, a compound of the formula (IV) having any one of the subformulae (IV-6)- (IV-17) is used in the above-mentioned step (A1) of the inventive method: 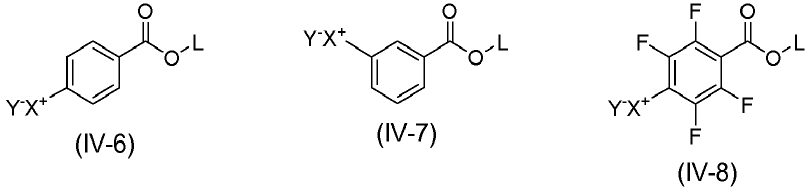 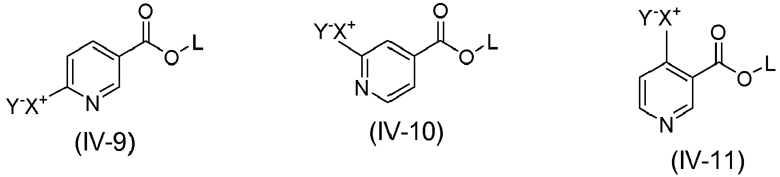 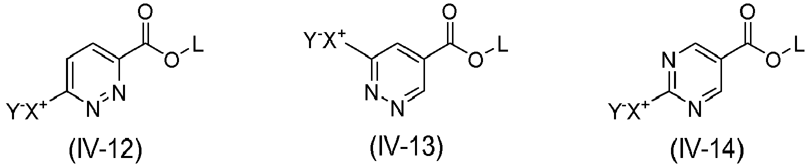 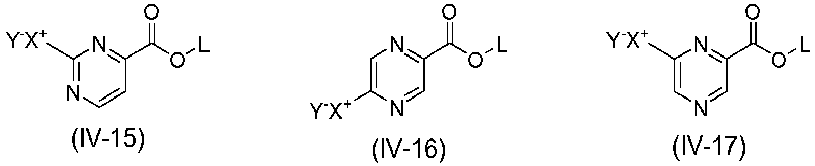 wherein L, X and Y have the same meanings as defined above.

Most preferred, a compound of the formula (IV) selected from the following compounds 4a - 4f is used in the above-mentioned step (A1) of the inventive method:

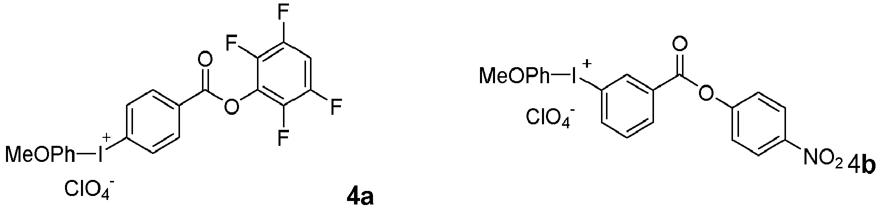

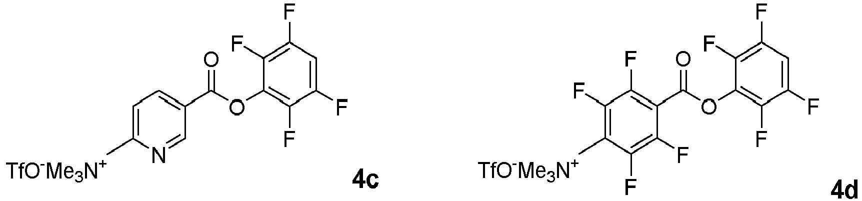

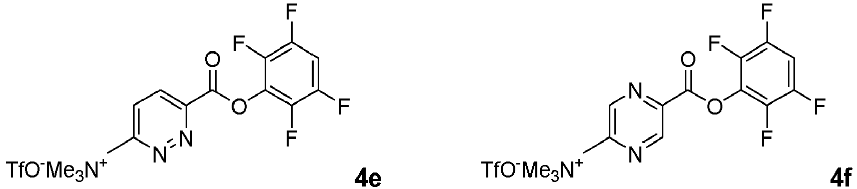

Preferred, a compound of the formula (III) having any of the subformulae (111-1) - (III-5) which derived from a compound of any of the subformulae (IV-1) - (IV-5), is obtained from the above-mentioned steps (A7) and (A8) or used in the above-mentioned step (B) of the inventive method: 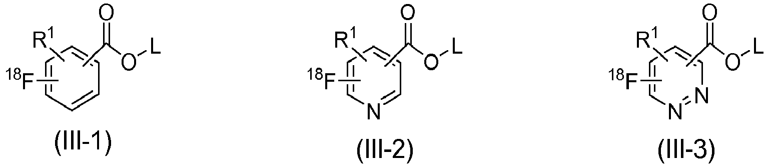 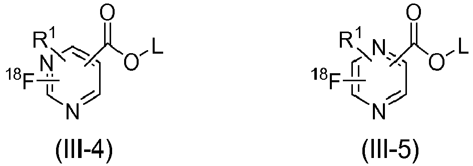 wherein L and R¹ have the same meanings as defined above.

Also preferred, a compound of the formula (III) having any of the subformulae (III-6)-(III-17) which derived from a compound of any of the subformulae (IV-6) - (IV-17), is obtained from the above-mentioned steps (A7) and (A8) or used in the above-mentioned step (B) of the inventive method: 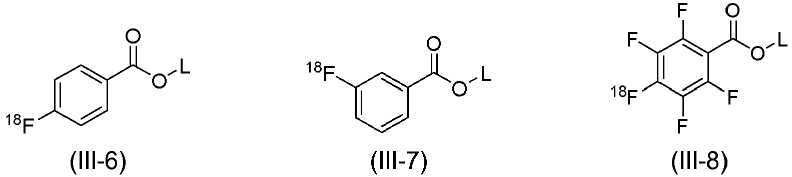 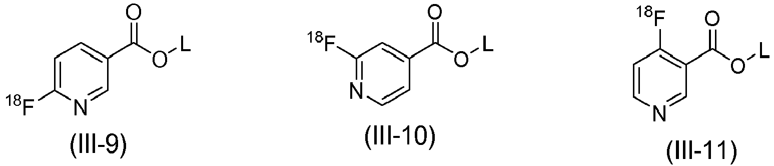 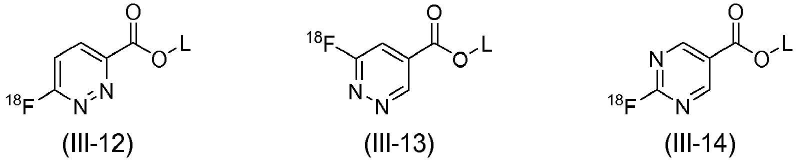 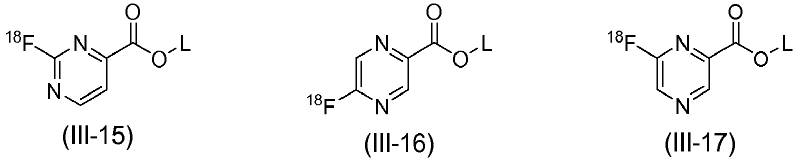 wherein L has the same meanings as defined above.

Most preferred, a compound of the formula **(III)** selected from the group consisting of the following compound **3a - 3f** derived from compounds **4a - 4f,** is obtained from the above-mentioned steps (A7) and (A8) or used in the above-mentioned step (B) of the inventive method:

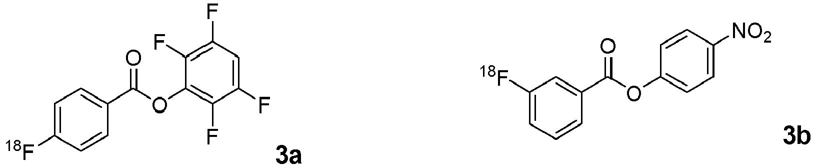

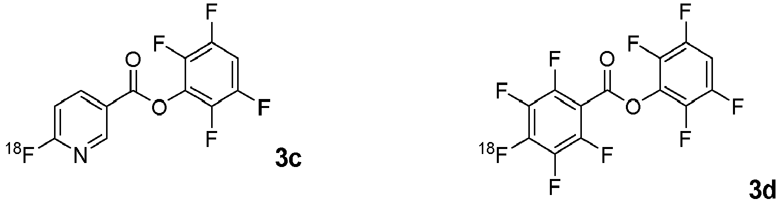

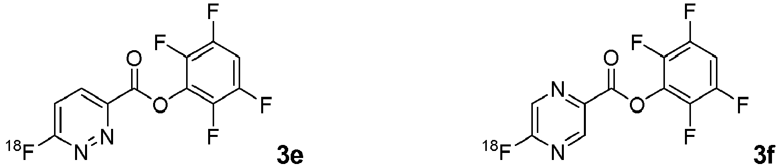

The term "C₁ - C₃ alkyl" as used herein refers to a saturated linear or branched carbon chain consisting of 1 to 4 carbon atoms. Examples are -CH₃, -C₂H₅, -C₃H₇, and -CH(CH₃)₂.

The term "C₁-C₃ alkoxy" as used herein refers to a C₁-C₃ alkyl group sigular bonded to oxygen and said C₁-C₃ alkyl has the same meaning as defined above. Examples are -OCH₃, -OC₂H₅, -OC₃H₇, -OCH(CH₃)₂.

The term "C₁-C₃ haloalkyl" as used herein refers to a saturated linear or branched carbon chain consisting of 1 to 4 carbon atoms substituted by at least one halogen atom such as F, Br, Cl and I. It is clear to a skilled person that the term "substituted" refers to the replacement of a hydrogen atom by one of the substituents. Examples of a C₁-C₃ haloalkyl are -CH₂F, -CHF₂, -CF₃, -CCl₃, -CH₂CF₃, -C₂F₅, -C₃F₇, -CH(CF₃)₂.

The term "C₁-C₃ haloalkoxy" as used herein refers to a C₁-C₃ haloalkyl group sigular bonded to oxygen and said C₁-C₃ haloalkyl group has the same meaning as defined above. Examples are -OCH₂F, -OCHF₂, -OCF₃, -OCCl₃, -OCH₂CF₃, -OC₂F₅, -OC₃F₇, -OCH(CF₃)₂.

The term "C₂-C₄ alkylcarbonyl" as used herein refers to a a C₁-C₃ alkyl group sigular bonded to carboxy group and said C₁-C₃ alkyl has the same meaning as defined above. Examples are -COCH₃, -COC₂H₅, -COC₃H₇, -COCH(CH₃)₂.

The term "C₂-C₄ alkylcarboxy" as used herein refers to a a C₁-C₃ alkyl group sigular bonded to carboxy group (-CO₂-) and said C₁-C₃ alkyl has the same meaning as defined above. Examples are -O₂CCH₃, -O₂CC₂H₅, -O₂CC₃H₇, -O₂CCH(CH₃)₂.

The term "C₂-C₄ alkyloxycarbonyl" herein refers to a a C₁-C₃ alkyl group sigular bonded to oxy carbonyl group (-O-CO-) and said C₁-C₃ alkyl has the same meaning as defined above. Examples are -CO₂CH₃, -CO₂C₂H₅, -CO₂C₃H₇, -CO₂CH(CH₃)₂.

The terms "aryl" as used herein refer to carbocyclic aromatic or heterocyclic aromatic residues or more specific to "C₆-C₁₀ carbocyclic aromatic residues with one or two aromatic rings and refers preferably to phenyl and naphthyl, wherein these aromatic or heteroaryl residues, preferred phenyl, thienyl and naphthyl residues can be substituted with 1 to 5 substituents selected from halogen like-F, -Br, -Cl, -I, C₁-C₃ alkyl, C₁-C₃ alkoxy, C₁-C₃ haloalkyl, C₁-C₃ haloalkoxy, -CN, -OH, -NO₂, -CO₂(C₁-C₃ alkyl), -NHCO(C₁-C₃ alkyl), -NH(C₁-C₃ alkyl), -N(C₁-C₃ alkyl)₂. It is clear to a skilled person that the term "can be substituted" refers to the replacement of a hydrogen atom by one of the said substituents. The carbon atom number of C₆-C₁₀ refers only to the carbon atoms of the aromatic ring system (aryl) and does not include the carbon atoms of the said substituents.

Examples are

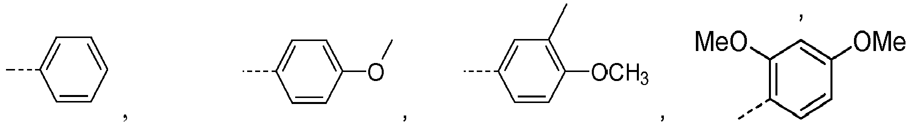

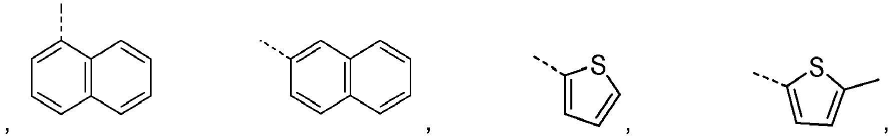

The term "aryloxy" as used herein refers to a aryl group sigular bonded to oxygen and said aryl has the same meaning as defined above.

The term "arylcarboxy" as used herein refers to a aryl group sigular bonded to carbonyl group (-CO₂-) and said aryl has the same meaning as defined above.

### ¹⁸F-Production

[¹⁸F]Fluoride was produced via the ¹⁸O(p,n)¹⁸F reaction by bombardment of enriched [¹⁸O]water with protons using a 16 MeV cyclotron.

Preferred, the [¹⁸F]fluoride is trapped on an anion exchange resin. The resin was washed with alcohol (e.g., with *n*PrOH, *i*PrOH, nBuOH, better with EtOH or MeOH) and drained with flow of gase (e.g., air, Ar, N₂, He). In the said method ¹⁸F⁻ is directly eluted with an alcoholic solution (e.g., in nPrOH, *i*PrOH, nBuOH, better in EtOH or MeOH) of the corresponding onium precursor (e.g., ONP or OTFP). Alternatively, ¹⁸F⁻ was eluted with an alcoholic solution (e.g., in *n*PrOH, *i*PrOH, nBuOH, better in EtOH or MeOH) of suitable salt, most preferably tetraethylammonium bicarbonate.

The term "C₁-C₄ alcohol" as used herein refers to a saturated linear, branched or cyclic C₁-C₄ alkyl group substituted by at least one hydroxyl group. It is clear to a skilled person that the term "substituted" refers to the replacement of a hydrogen atom by one of the substituents. Examples of a C₁-C₄ alcohol are CH₃OH, CH₃CH₂OH, HOCH₂CH₂OH, CH₃CH₂CH₂OH, HOCH₂CH₂CH₂OH, HOCH₂CH(OH)CH₃, HOCH₂CH(OH)CH₂OH, (CH₃)₂CHOH, CH₃CH₂CH₂CH₂OH, CH₃CH₂CH(OH)CH₂OH, CH₃CH(OH)CH₂CH₂OH, CH₂(OH)CH₂CH₂CH₂OH, CH₂(OH)CH(OH)CH₂CH₂OH, CH₃CH(OH)CH(OH)CH₂OH, CH₂(OH)CH(OH)CH(OH)CH₂OH, (CH₃)₂CHCH₂OH, H₃CCH(CH₂OH)₂, HC(CH₂OH)₃, and (CH₃)₃C-OH. Preferred, CH₃OH or CH₃CH₂OH is used as the C₁-C₄ alcohol in the above-described method.

The term "C₃-C₆ alcohol" as used herein refers to a saturated linear, branched or cyclic C₃-C₆ alkyl group substituted by at least one hydroxyl group. It is clear to a skilled person that the term "substituted" refers to the replacement of a hydrogen atom by one of the substituents. Preferred examples of a C₃-C₆ alcohol are CH₃CH₂CH₂OH, (CH₃)₂CHOH, CH₃CH₂CH₂CH₂OH, (CH₃)₂CHCH₂OH, (CH₃)₃C-OH, CH₃CH₂CH₂CH₂CH₂OH, (CH₃)₂CHCH₂CH₂OH, CH₃CH₂CH₂CH₂CH₂CH₂OH, (CH₃)₂CHCH₂CH₂CH₂OH, HOCH₂CH₂CH₂OH. In an embodiment of the present invention, t-BuOH ((CH₃)₃C-OH) is used as the C₃-C₆ alcohol in the above-described method.

The term "aprotic solvent" or "polar aprotic solvent" as used herein refers to acetone, DMF, DMA, 1,2-dimethoxyethane (glyme), , MeCN, 2-methoxyethyl ether (diglyme), *N*-methylpyrrolidinone, DMSO, sulfolane, propylenecarbonate, or THF. Preferred, MeCN, and DMSO are used.

The term "polar protic solvent" as used herein refers to any solvent that contains labile H⁺ and readily donate protons (H⁺) to reagents and capable ot dissolve the salts. Preferred examples of a polar protic solvent are MeOH, EtOH, n-PrOH, *i-*PrOH, n-BuOH, HOCH₂CH₂OH, HOCH₂CH₂CH₂OH, HOCH₂CH(OH)CH₃, HOCH₂CH(OH)CH₂OH, HC(CH₂OH)₃, HO-C₂H₄-O-C₂H₄-OH, HO-C₂H₄-O-C₂H₄-O-C₂H₄-OH, HO-C₂H₄-O-C₂H₄-O-C₂H₄-O-C₂H₄-OH, HCO₂H and CH₃CO₂H.

In an embodiment of the present invention, EtOH is used as the polar protic solvent in the above described method.

The term "RCY (radiochemical yield)" as used herein refers to the yield for the step in which the radiolabel is introduced. The RCY refers to the activity (decy-corrected) of the radiolabel led product expressed as a fraction of the activity originally present.

Preferred is said method for preparing a compound of any of the formulae (**Ia**)-(**Ie**): 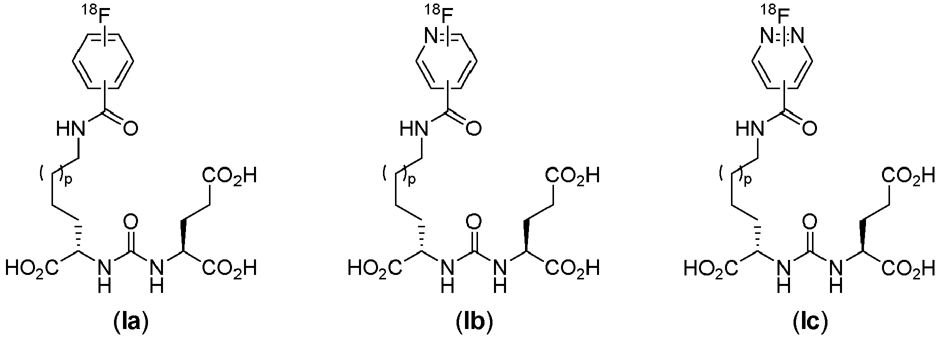 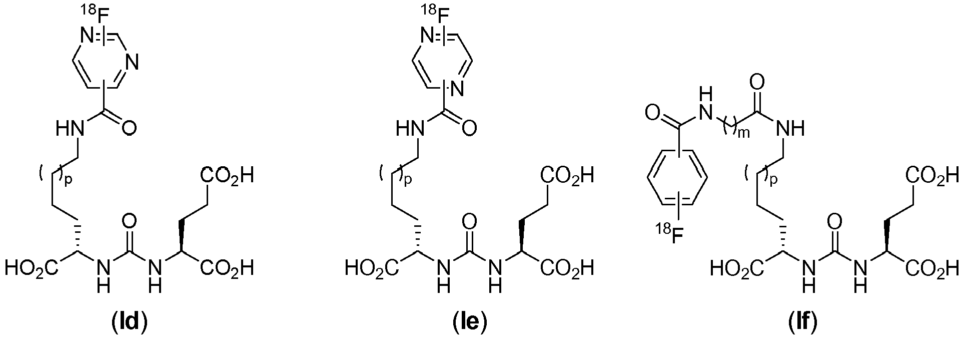 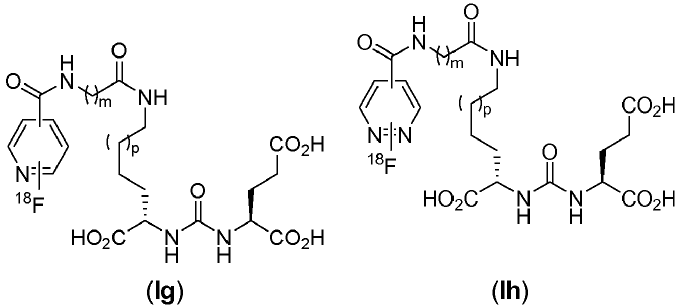 wherein m is an integer from 1 to 8; **p** is an integer from 0 to 2.

Also preferred is said method for preparing a compound of the formula (I) selected from the compounds of the following subformulae (1-1) - (I-6):

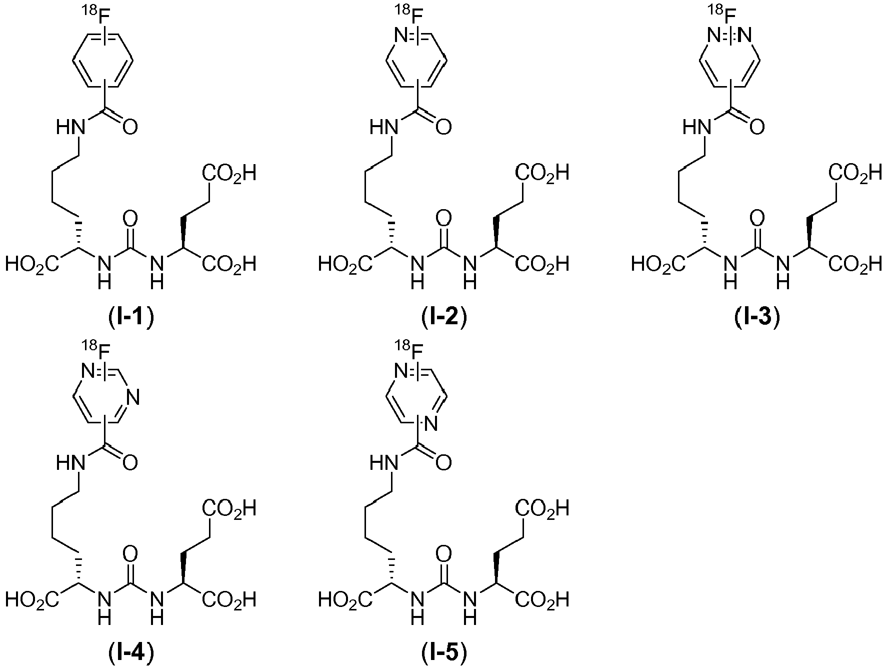

More preferred is said method for preparing a compound of the formula (I) selected from the compounds of the following subformulae **(I-6) - (I-15):**

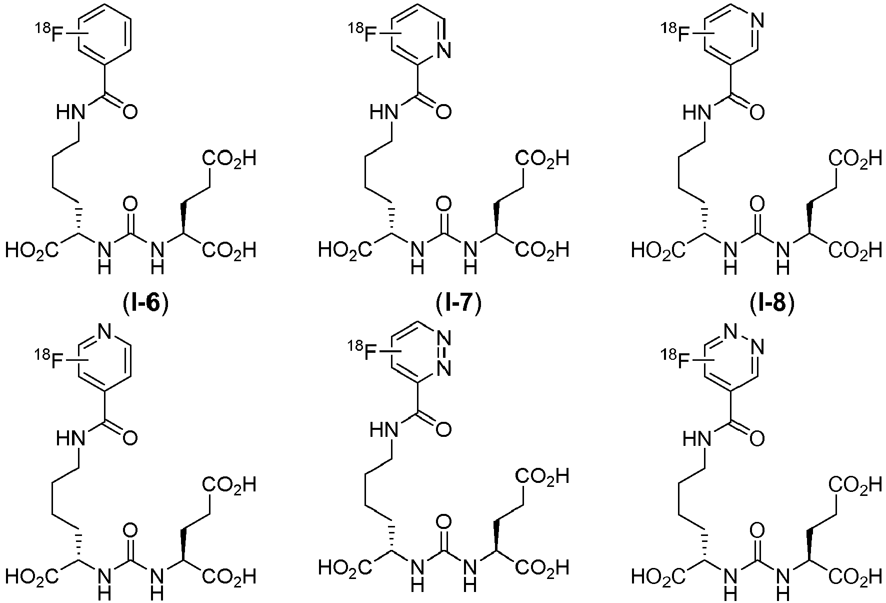

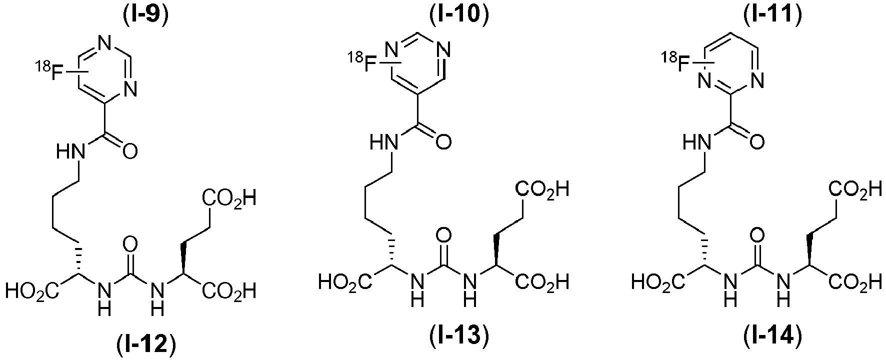

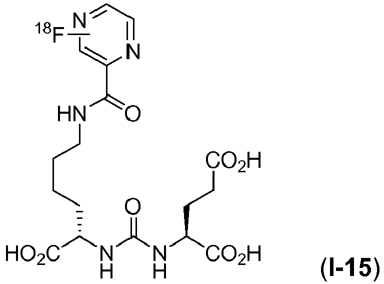

Still more preferred is said method for preparing a compound of the formula (I) selected from the following compounds **1-1 - 1-34.**

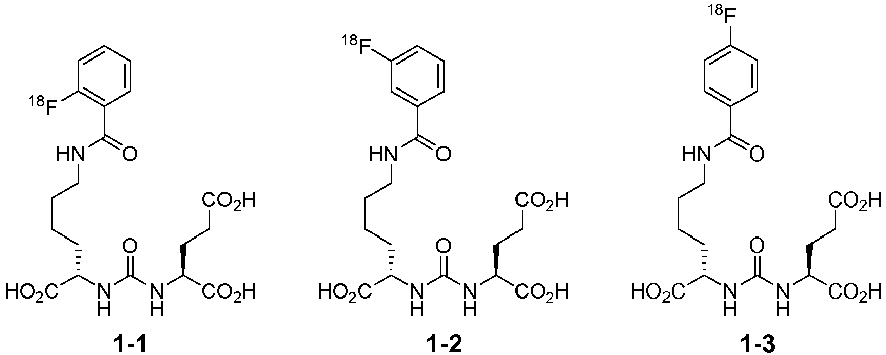

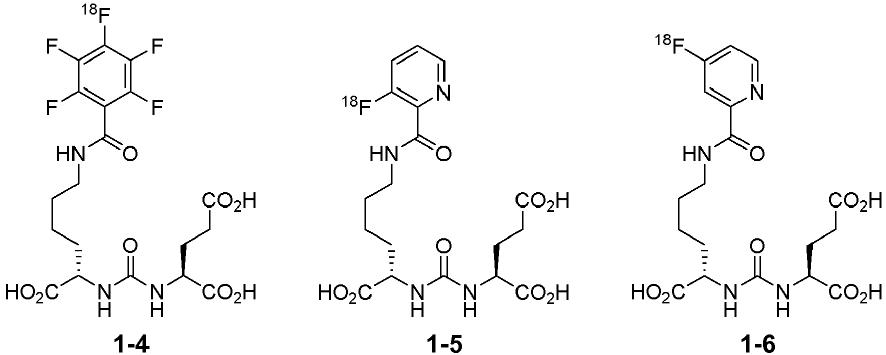

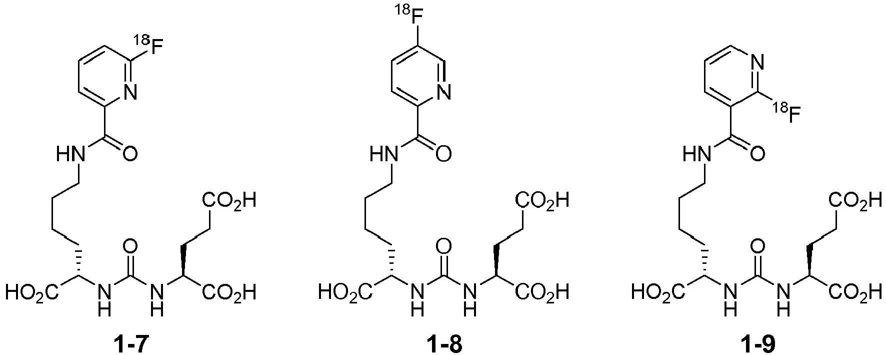

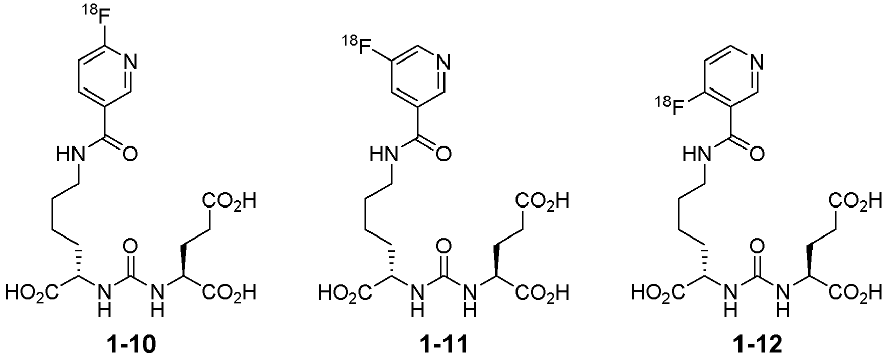

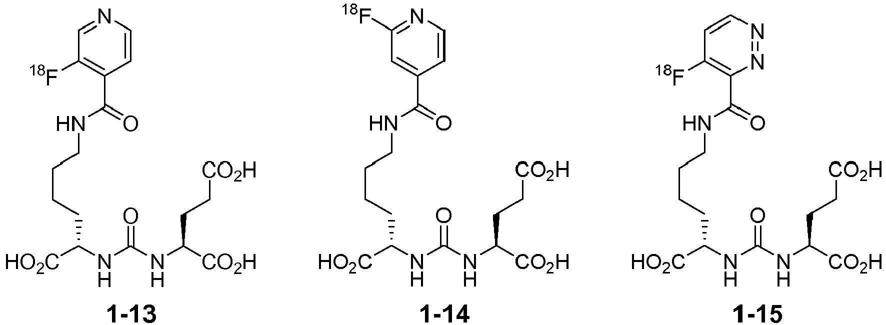

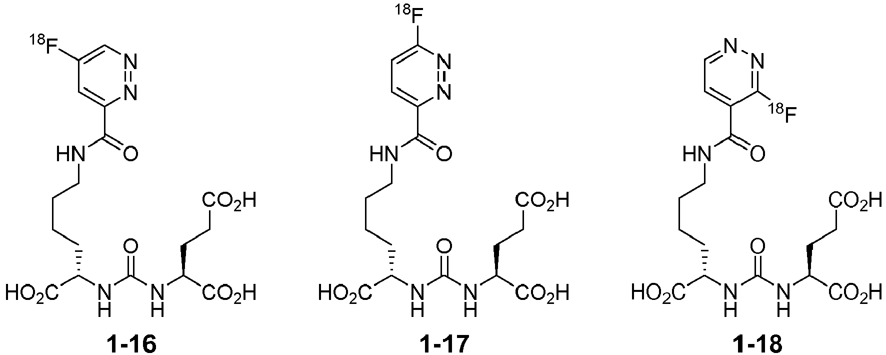

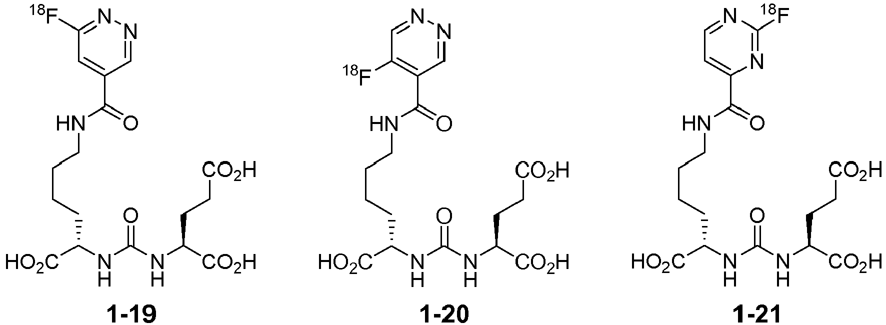

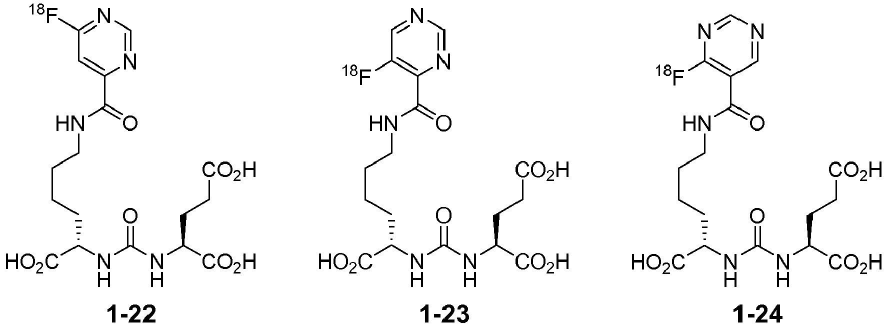

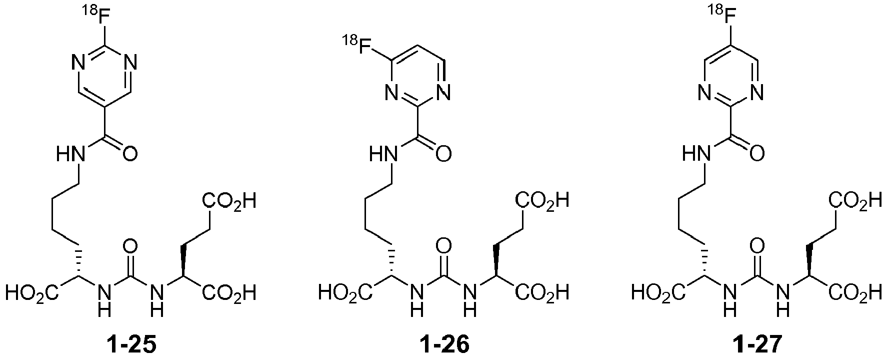

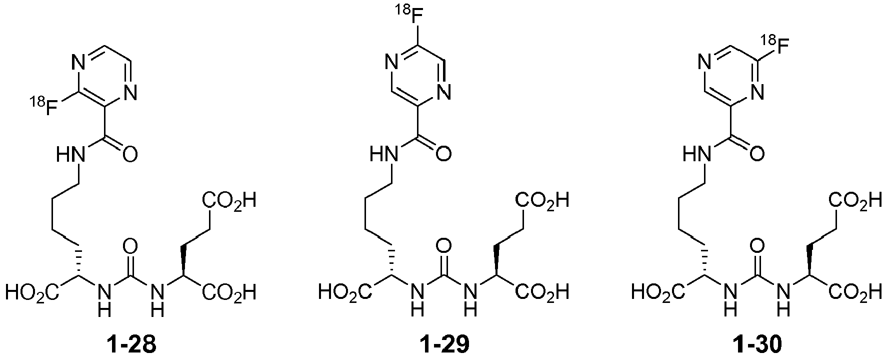

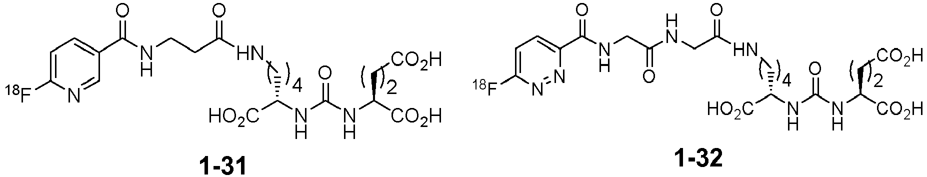

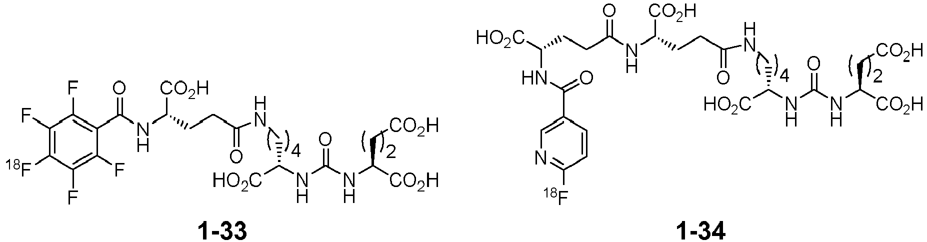

Most preferred is said method for preparing the compound of the formula (I) selected from the group consisting of compounds **1-3, 1-10, 1-14, 1-17, 1-29, 1-31, 1-32, 1-33,** and **1-34:** 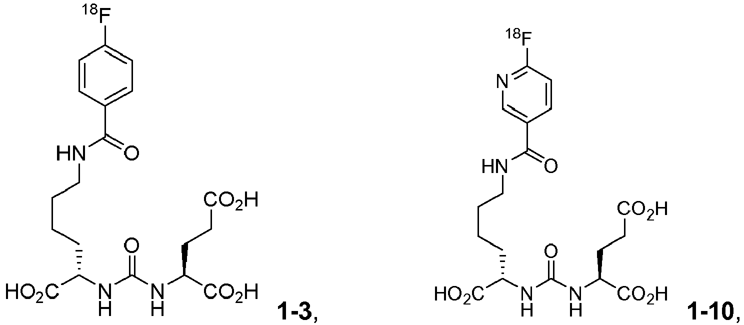 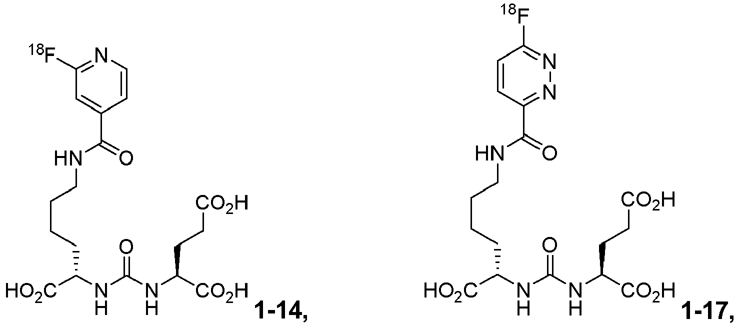 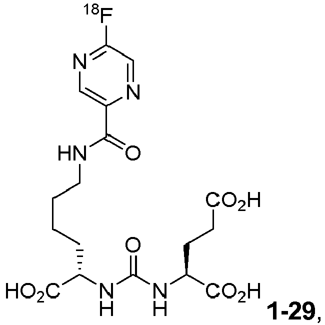 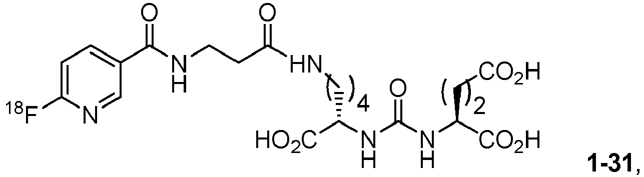 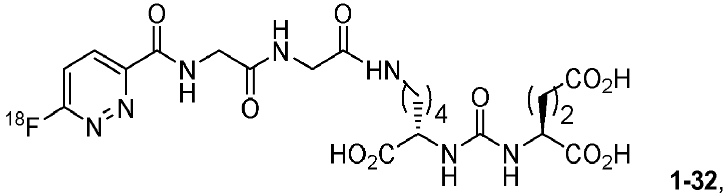 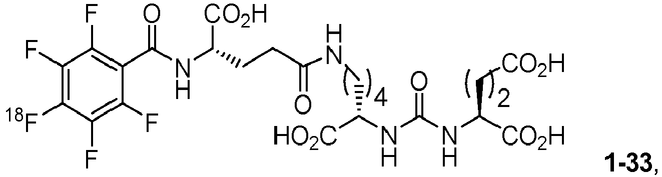 and

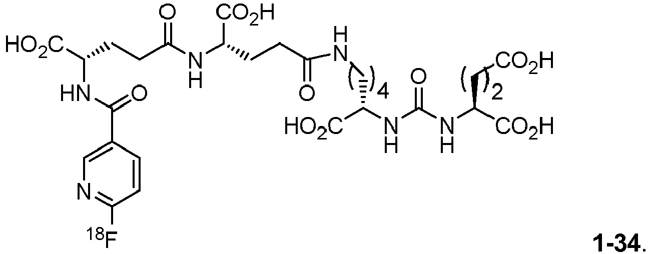

The present invention refers to the compound of the formula (**I**) obtainable by any of the above mentioned inventive methods, preferably the compound of any of the subformulae (**Ia**) - (**Ie**) and (**I-1**) - (**I-15**) obtainable by any of said inventive methods, more preferably the compounds **1**-**1** - **1-34** obtainable by any of said inventive methods. 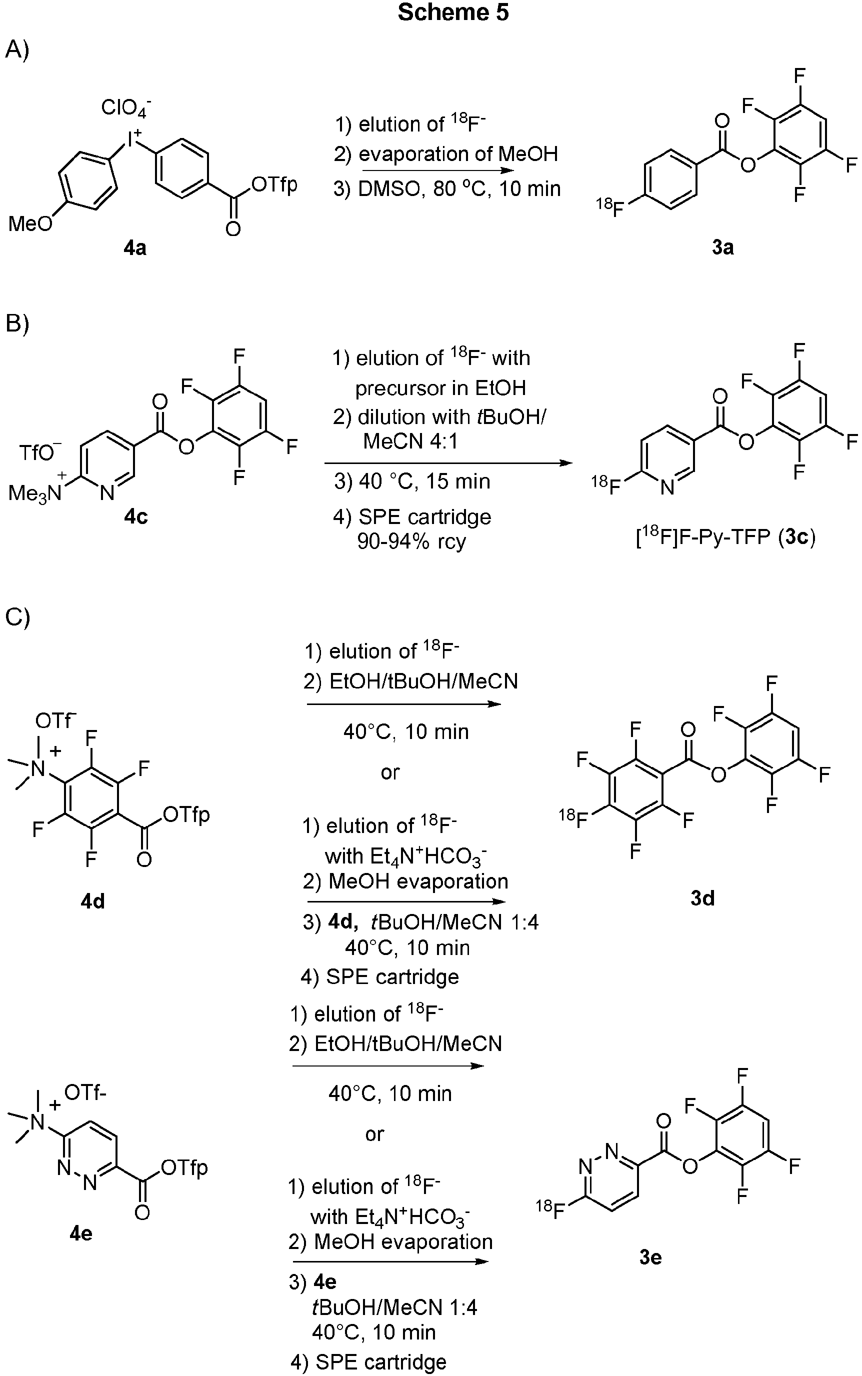 In the present invention, compound of the formula (III) as activated aromatic ester (pyridine, pyrazine, pyrimidine, tri/tetrahalo, cyano-, nitro-, (per)fluoralkyl-, like trifluoromethyl-, alkylcarboxybenzene or similar) can be prepared by SN_{Ar} radiofluorination of precursor compound of the formula (IV) as shown in Scheme 5.

In case of less activated precursors of the formulae IV-6 and IV-7, e.g., (4-methoxyphenyl)[4-(2,3,5,6-tetrafluorophenoxycarbonyl)phenyl]iodanium perchlorate 2,3,5,6-tetrafluorophenyl 4-(4-methoxyphenyl)phenyliodonium benzoate perchlorate (**4a**), the solvent should be removed under reduced pressure before the nucleophilc reaction can be carried out. The residue is taken up in a suitable solvent and subsequently heated for a short time to give the desired radidolabelled active ester as shown Scheme 5A.

In case of highly activated precursors of the formulae IV-8 - IV-17, e.g. *N,N,N-*trimethyl-5-[(2,3,5,6-tetrafluorophenoxy)-carbonyl]pyridine-2-aminium trifluoromethanesulfonate (**4c**), removal of alcohol (except MeOH) is unnecessary. Accordingly, the ¹⁸F-eluate can be simply diluted with the corresponding solvent (e.g., *t*BuOH, *t*BuOH/MeCN, MeCN, etc.) and directly heated for a short time. Finally, the radiolabelled active esters are isolated using HPLC or SPE. In both cases neither azeotropic drying nor base nor any other additives are necessary (Scheme 5B).

The described methods allow to prepare e. g., [¹⁸F]TFB **3a** and [¹⁸F]F-Py-TFP 3c in 20-25% and 90-94% RCYs, respectively.

Alternatively, ¹⁸F-labelled active esters from highly activated precursors can be prepared using alcoholic tetraethylammonium bicarbonate solutions (e.g., in *n*PrOH, *i*PrOH, *n*BuOH, better in EtOH or MeOH) for ¹⁸F⁻ elution. The eluate is concentrated under reduced pressure. Precursor for radiolabelling in a suitable solvent (e.g., *t*BuOH, *t*BuOH/MeCN, MeCN, etc.) is added and the reaction mixture is heated for a short time to give the respective active ester in more than 90% RCY. The building block is isolated via RP-SPE in excellent radiochemical and chemical purity (Scheme 5C).

### Application of the radiolabelled active esters (III) for the preparation of PET tracers

An application of the ¹⁸F-labelled active esters synthesized according to the inventive procedures is exemplified by the simplified preparation of the PSMA-specific tracer [¹⁸F]DCFPyL **1-10.**

According to the inventive method, [¹⁸F]F-Py-TFP 3c obtained under "nothing added conditions" was eluted from the resin with ethanol in a solution of HO-Lys-C(O)-Glu-OH 2b and tetraethylammonium bicarbonate in ethanol (Scheme 6). The reaction mixture was heated at 40°C for 3 min. [¹⁸F]DCFPyL 1-10 was isolated by RP-HPLC using 10% ethanol in isotonic saline as an eluent to give after sterile filtration the ready to use solution of the radiotracer.

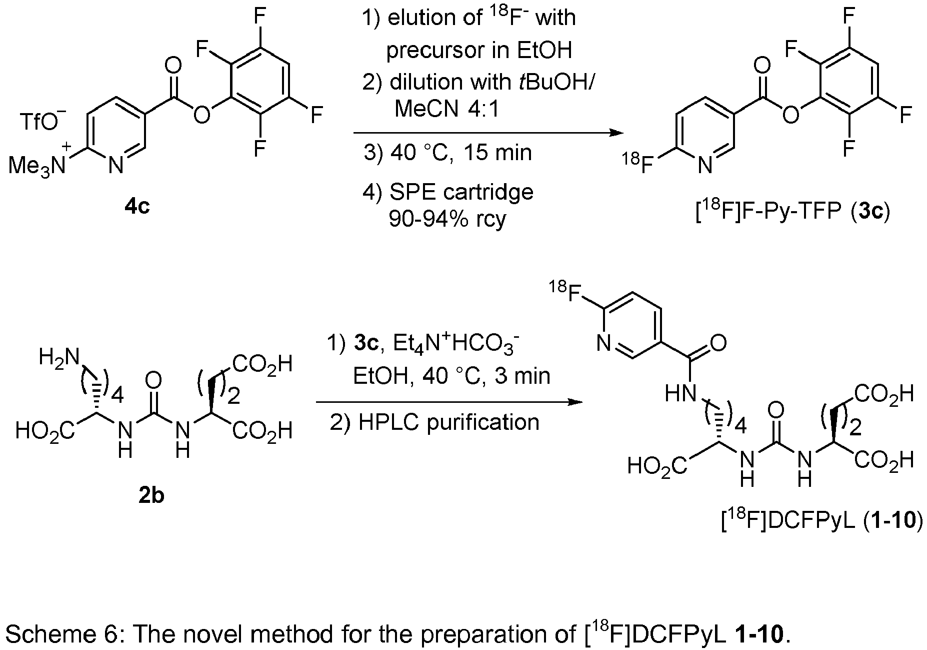

In the inventive method ¹⁸F⁻ is trapped on an anion exchange resin and then eluted directly with an alcoholic solution of the radiolabelling precursor containing an onium group (X) like R²₃N⁺, Arl⁺ or Ar₂S⁺. The solvent is removed under reduced pressure, the residue is taken up in a suitable solvent and heated for a short time. In case of highly activated radiolabelling precursors such as the precursor of [¹⁸F]F-Py-TFP **3c** solvent removal is unnecessary. In this case an alcoholic solution (except MeOH) of the onium [¹⁸F]fluoride salt is simply diluted with a suitable solvent and heated. The purification/isolation of the radiolabelled active esters is accomplished by SPE or HPLC purification. Neither azeotropic drying nor a base nor any other ingredients are needed. Alternatively, elution of radiofluoride can be accomplished with tetraethylammonium bicarbonate (TEABC) in MeOH. In this case neither azeotropic drying nor any other ingredients are needed.

The inventive modifications of the common radiofluorination procedure allow a considerable shortening of the overall preparation time, due to the reduction of a number of preparation steps. Additionally radiofluorination according to Methods 1 and 2 is carried out under base free conditions that enables one step preparation of the ¹⁸F-labelled active esters even from less activated labelling precursors where harsher reaction conditions (higher temperatures, longer reaction times) are necessary. These advantages allow to carry out the radiosynthesis in one pot making the inventive methods well-suited for automated radiosyntheses, especially in microfluidic devices.

In contrast to the published radiosynthesis of [¹⁸F]DCFPyL **1-10** by Pomper et al. the inventive method for the preparation of this radiotracer allows to omit two evaporation steps, application of water immiscible solvents (CH₂Cl₂) as well as toxic reagents (TFA, anisole, MeCN, CH₂Cl₂) the final deprotection and additional formulation steps.

The inventive method enables automatic synthesis of [¹⁸F]DCFPyL **1-10** as shown in Example 3.

Consequently, using the inventive method [¹⁸F]DCFPyL **1-10** could be prepared faster and in higher isolated RCYs of up to 45% over 2 steps within only 55 min after end of bombardment (EOB) compared to RCYs of 35% over 3 steps within 128 min EOB. The automatic synthesis yields [¹⁸F]DCFPyL **1-10** in isolated RCYs up to 35% in only 50 min.

### Pharmaceuticl composition

Another aspect of the present invention relates to pharmaceutical compositions comprising at least one compound of formula (I) together with at least one pharmaceutically acceptable solvent, ingredient and/or diluents. Said pharmaceutical composition is useful for imaging prostate cancer cells or prostate cancerous tissue.

Pharaceutically acceptable ingredient refers to especially antiviral, antibacterial or antifungal agents.

Diluent includes any one of sterile saline, sodium chloride injection, Ringer's injection, dextrose injection, dextrose and sodium chloride injection, lactated Ringer's injection and other aqueous buffer solution. The amount of diluent in the composition can range from about 5 to about 95 % by weight of the total composition, preferably from about 25 to about 75 weight %, and more preferably from about 30 to about 60 weight %.

The inventive pharmaceutical composition may be administered by injection (intravenous, intraperitoneal, intramuscular, subcutaneous).

### PET Imaging

So far the only PSMA PET tracer used in clinics is [⁶⁸Ga]HBED-CC **(50).** ⁶⁸Ga has a half-life of 68 min and is obtained from a generator system in only moderate amounts that precludes the broad application of [⁶⁸Ga]HBED-CC **(50)** in clinical practice. In contrast, ¹⁸F has the advantage of a longer half-life (110 min) and is accessible in high amounts of up to 74 GBq or more. This permits the centralized production and regional distribution as practiced in the supply of [¹⁸F]FDG for clinical use. In a suitable PSMA⁺ PCa mice model [¹⁸F]DCFPyL **1-10** displays imaging characteristics nearly identical to those of [⁶⁸Ga]HBED-CC **(50).** Moreover, kidney uptake of [¹⁸F]DCFPyL **1-10** is much lower as that of [⁶⁸Ga]HBED-CC **(50).** This should improve the detection of tumor lesions in the abdomen. Therefore, taking into an easy accessibility of [¹⁸F]DCFPyL **1-10** according to the present invention it could represents an adequate alternative for [⁶⁸Ga]HBED-CC **(50)** for research and patient care.

### Description of Figures

- **Figure 1:**: Automatic synthesis of [¹⁸F]DCFPyL in TRACERLab FX_{fn} automated radiochemistry synthesis module (GE Medical Systems).
- **Figure 2:**: [⁶⁸Ga]HBED-CC- **(50)** versus [¹⁸F]DCFPyL (**1-10**)-PET in PSMA⁺ PCa bearing mice. Shown are measurements in the same animal, on two consecutive days. Cursor position is indicated by crosslines. Abbreviations: K: kidney; T: tumor.Scale bar: 10 mm.

### Examples

### Abbreviations:

ßAla (beta-alanine), Boc (tert-butyloxycarbonyl), DCC (N,N'-dicyclohexylcarbodiimide), DMF (dimethylformamide), DMM (dimethoxymethane), DMSO (dimethyl sulfoxide), EDC (1-ethyl-3-(3-dimethylaminopropyl)carbodiimide), EDTA (ethylenediaminetetraacetic acid), Glu (glutanic acid), Gly (glycine), Lys or syL (lysine) mCPBA (meta-chloroperoxy benzoic acid), MeCN (acetonitrile), ONSu (N-oxy-succinimide), OTf (trifluoromethanesulfonate), Tos (tosyl), PMB (p-methoxybenzyl), Py (pyridinyl), tBu (tert-butyl), TFA (trifluoroacetic acid), TFE (tetrafluoroethyl alcohol), TFP (2,3,5,6-tetrafluorophenyl), THF (tetrahydrofuran), TIS (triisopropylsilane), TMS (trimethylsilyl).

### Example 01: Preparation of 2,3,5,6-Tetrafluorophenyl-6-[¹⁸F]fluoronicotinate ([¹⁸F]F-Py-Tfp, 3c).

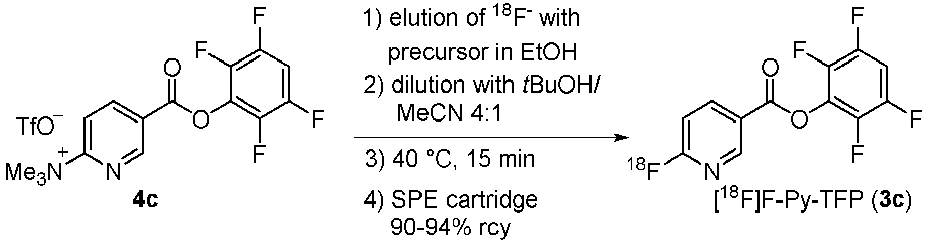

**Procedure A:** Aqueous [¹⁸F]fluoride (0.05-50 GBq) was trapped on a anion-exchange resin (QMA or Chromafix^{®} 30-PS-HCO₃ cartridge). It should be noted, that in the case of QMA cartridges, the aqueous [¹⁸F]fluoride was loaded onto the cartridge from the male side, whereas EtOH flushing and ¹⁸F⁻ elution were done from the female side of the cartridge. The cartridge was washed with EtOH (1 mL) and [¹⁸F]fluoride was eluted from the resin into the reaction vial with *N,N,N-*trimethyl-5-[(2,3,5,6-tetrafluorophenoxy)-carbonyl]pyridine-2-aminium trifluoromethanesulfonate **4c** (10 mg, 20 µmoL) in EtOH (200 µL) followed by MeCN/*t*BuOH 1:4 (2 mL). The mixture was stirred for 15 min at 40 °C. After cooling to ambient temperature, the reaction mixture was diluted with water (20 mL) and loaded onto a polymer RP cartridge (the cartridge was preconditioned with 2 mL EtOH followed by 30 mL H₂O). The cartridge was washed H₂O (5 mL) and [¹⁸F]F-Py-Tfp **3c** (up to 15 GBq, 70-75% EOB; not decay corrected) was eluted with EtOH (300 µL). The radiochemical and chemical purities after SPE purification were > 98% determined by analytical HPLC (Eluent: Water with 50% MeCN. Flow rate: 1.5 mL/min. Column: Chromolith^{®} SpeedROD RP-18e column (Merck, Darmstadt Germany), 50x4.6 mm. Retention time: [¹⁸F]F-Py-Tfp **3c** ∼ 2 min).

### Procedure B:

Aqueous [¹⁸F]fluoride (0.05-50 GBq) was trapped on a anion-exchange resin (QMA or Chromafix^{®} 30-PS-HCO₃ cartridge). It should be noted, that in the case of QMA cartridges, the aqueous [¹⁸F]fluoride was loaded onto the cartridge from the male side, whereas EtOH flushing and ¹⁸F⁻ elution were done from the female side of the cartridge. [¹⁸F]fluoride was eluted from the resin into the reaction vial with methanolic tetraethylammonium bicarbonate solution (5 mg, 500 µL). The solvent was removed under reduced pressure at 70 C within 2 min. Afterwards precursor dissolved in MeCN/*t*BuOH 1:4 (2 mL) was added and the mixture was stirred for 15 min at 40 °C. After cooling to ambient temperature, the reaction mixture was diluted with water (20 mL) and loaded onto a polymer RP cartridge (the cartridge was preconditioned with 2 mL EtOH followed by 30 mL H₂O). The cartridge was washed H₂O (5 mL) and [¹⁸F]F-Py-Tfp 3c (up to 15 GBq, 70-75% EOB; not decay corrected) was eluted with EtOH (300 µL). The radiochemical and chemical purities after SPE purification were > 98% determined by analytical HPLC (Eluent: Water with 50% MeCN. Flow rate: 1.5 mL/min. Column: Chromolith^{®} SpeedROD RP-18e column (Merck, Darmstadt Germany), 50x4.6 mm. Retention time: [¹⁸F]F-Py-Tfp **3c** ∼ 2 min).

### Example 02: Preparation of [¹⁸F]DCFPyL 1-10

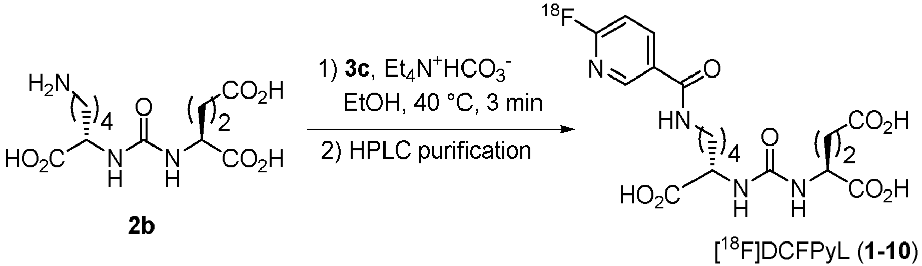

To a freshly prepared ethanolic solution of HO-Lys-C(O)-Glu-OH **2b** (2.5 mg, 100 µL) and tetraethylammonium bicarbonate (0.5 M) in EtOH (60 µL) an ethanolic solution of [¹⁸F]F-Py-Tfp **3c** (0.05-10 GBq in 150 µL) was added. The reaction mixture was stirred for 3 min at 40 °C. Afterwards the mixture was quenched with water (2 mL) and purified by preparative HPLC. Eluent: saline (0.9% NaCl) with 10% EtOH. Flow rate: 8 mL/min. Column: Synergi 4µm Hydro-RP 80A 100×21,2 mm. Retention time: ∼7 min. The purified product was sterile filtered before use. [¹⁸F]DCFPyL **1-10** was obtained in 75-90% RCY (decay-corrected). The radiochemical and chemical purities after HPLC purification were > 98%, determinded by analytical HPLC: Eluent: phoshoric acid buffer solution (pH = 2) with 10% EtOH for 5 min, then 50% for 2 min. Flow rate: 1.5 mL/min. Column: Chromolith^{®} SpeedROD RP-18e column (Merck, Darmstadt Germany), 50x4.6 mm. Retention times: [¹⁸F]DCFPyL ∼3 min; [¹⁸F]F-Py-Tfp ∼ 5.7 min).

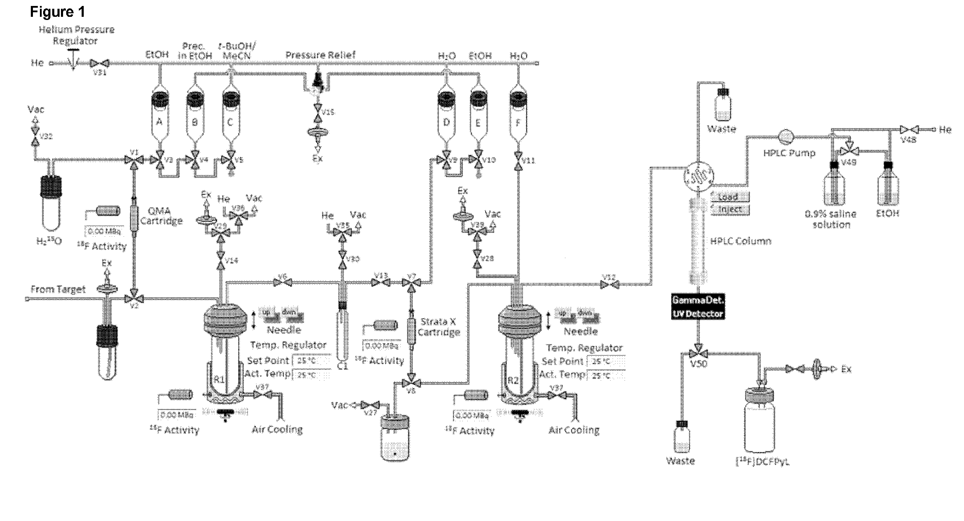

### Example 03: Automated synthesis of [¹⁸F]DCFPyL 1-10 starting from [¹⁸F]fluoride (Figure. 1)

Aqueous [¹⁸F]fluoride (0.05-50 GBq) was vacuum-transferred from the target to a trapping vial. Aqueous [¹⁸F]fluoride was then transferred from the trapping vial through an anion-exchange resin cartridge (QMA) from the male side of the cartridge, and [¹⁸O]H₂O was collected in a separate vial. The cartridge was subsequently washed with EtOH (1 mL) from vial **A** from the female side of the cartridge. Washings were discarded. Thereafter, [¹⁸F]fluoride was eluted from the resin with *N,N,N*-trimethyl-5-[(2,3,5,6-tetrafluorophenoxy)-carbonyl]pyridine-2-aminium trifluoromethanesulfonate **4c** (10 mg, 20 µmoL) in EtOH (200 µL) from vial **B** into reactor **R1.** After this, MeCN/*t*BuOH 1:4 (2 mL) from vessel **C** was passed through the cartridge into reactor **R1.** Reactor **R1** was filled with helium, sealed and the reaction mixture was heated at 45 °C for 20 min. After cooling to room temperature the crude [¹⁸F]F-Py-Tfp **3c** was transferred into the collecting vial **C1,** containing 20 mL H₂O. The solution was passed through a polymer RP cartridge (Strata X). The cartridge was washed with H₂O (5 mL) from reservoir **D** and dried by applying a helium stream for 5 min. [¹⁸F]F-Py-Tfp **3c** was eluted with EtOH (400 µL) from vial **E** into reactor **R2** containing a mixture of a freshly prepared ethanolic solution of HO-syL-C(O)-Glu-OH **2b** (2.5 mg, 100 µL) and 0.5 M tetraethylammonium bicarbonate in EtOH (60 µL). After addition of [¹⁸F]F-Py-Tfp, the reaction mixture was heated at 40 °C for 3 min. After cooling to room temperature the reaction mixture was diluted with H₂O (2 mL) from vial **F** and [¹⁸F]DCFPyL was loaded onto the HPLC for purification. The crude tracer was purified by preparative HPLC to give [¹⁸F]DCFPyL **1-10** in 40-65% RCY (from ¹⁸F⁻ ; decay-corrected) in ≥95% radiochemical purity. Eluent: saline (0.9% NaCl) with 10% EtOH. Flow rate: 8 mL/min. Column: Synergi 4µm Hydro-RP 80Å 100×21,2 mm. Retention time: ∼7 min. The purified product was sterile filtered before use (determinded by analytical HPLC: Eluent: phoshoric acid buffer solution (pH = 2) with 10% EtOH for 5 min, then 50% for 2 min. Flow rate: 1.5 mL/min. Column: Chromolith^{®} SpeedROD RP-18e column (Merck, Darmstadt Germany), 50x4.6 mm. Retention times: [¹⁸F]DCFPyL ∼3 min; [¹⁸F]F-Py-Tfp ∼ 5.7 min).

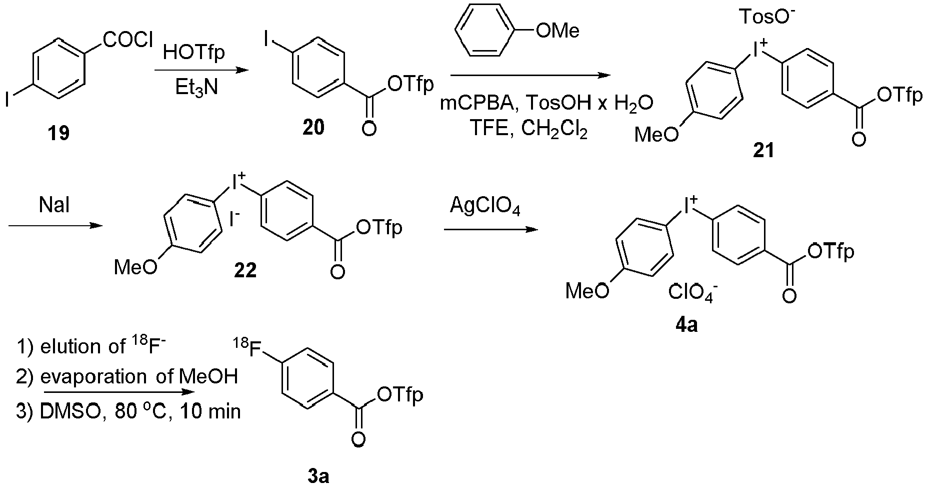

### Example 04: Preparation of 2,3,5,6-tetrafluorophenyl 4-iodobenzoate 20

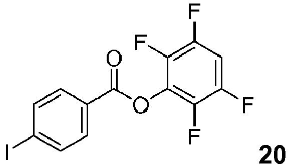

Et₃N (1.504 mL, 0.76 g, 7.51 mmol) was added dropwise to a vigorously stirred solution of 4-iodobenzoyl chloride 19 (2 g, 7.51 mmol) and 2,3,5,6-tetrafluorophenol (1.25 g, 7.51 mmol) in Et₂O (60 mL) and the stirring was continued for a further 10 min. The reaction mixture was filtered, the filter cake was washed with Et₂O (30 mL) and the filtrate was concentrated under reduced pressure. The residue was dissolved Et₂O (10 mL) and filtered. The filtrate was concentrated under reduced pressure. The residue was recrystallized from Et₂O/hexane to give the title compound (1.38 g, 48%) as a colorless solid.
*R*_{f} = 0.46, EtOAc:hexane = 1:10.
¹H-NMR(CDCl₃, 300 MHz): δ = 7.07 (tt, J = 9.9, 7.1 Hz, 1 H), 7.82-7.98 (m, 4 H); ¹⁹F-NMR (CDCl₃, 282.4 MHz): δ = -152.70, -138.80;
¹³C-NMR(CDCl₃, 75.5 MHz): δ = 103.0, 103.4 (t, *J* = 23.0 Hz), 126.6, 131.9, 138.3, 138.9-142.7 (m), 144.4 (dt, *J* = 3.8, 12.1 Hz), 147.7 (dt, *J* = 4.5, 12.1 Hz), 162.2. MS (ESI): positive mode *m*/*z* = 397.3 ([M + H]⁺). MS (El, 70 eV): m/z (%): 395.9 [M⁺] (3), 230.9 [C₇H₄OI⁺] (100), 202.9 [C₆H₃I⁺] (100), 104.0 [C₇H₄O⁺] (10).

### Example 05: Preparation of (4-Methoxyphenyl) [4-(2,3,5,6-tetrafluorophenoxycarbonyl)phenyl]iodonium tosylate 21:

Tos·H₂O (0.72 g, 3.79 mmol) was added to a solution of 2,3,5,6-tetraphenyl 4-iodobenzoate **20** (1 g, 2.52 mmol), mCPBA [1.44 g, 85% purity, 7.09 mmol; commercially available 77% mCPBA (Aldrich) was dried at 2 mbar and 40 °C for 3 h before use] and anisole (0.51 mL, 0.51 g, 4.72 mmol) in 50% CF₃CH₂OH (TFE) in CH₂Cl₂ (20 mL) and the mixture was stirred for 3 days. The reaction mixture was added to vigorously stirred Et₂O (450 mL) and stirring was continued for a further 45 min. The precipitate was filtered off and washed with Et₂O (100 mL), redissolved in CH₂Cl₂ (20 mL) and filtered through Celite^{®}. The filtrate was concentrated under reduced pressure. The residue was recrystallized from CH₂Cl₂/Et₂O to give the title compound (1.53 g, 90%) as a colorless solid.
¹H-NMR(CDCl₃, 300 MHz): δ = 2.28 (s, 3 H), 3.77 (s, 3 H), 6.80 (d, *J* = 9.0 Hz, 2 H), 6.97 (d, *J* = 6.0 Hz, 2 H), 7.01-7.14 (m, 1 H), 7.36 (d, *J* = 9.0 Hz, 2 H), 7.98-8.03 (m, 4 H), 8.18 (d, *J=* 9.0 Hz, 2 H);
¹⁹F-NMR (CDCl₃, 282.4 MHz): δ= -152.70, -138.55;
¹³C-NMR(CDCl₃, 75.5 MHz): δ = 21.2, 55.5, 103.7 (t, *J =* 23.0 Hz), 104.7, 117.4, 123.0, 125.9, 128.5, 129.3, 132.7, 135.3, 137.9, 138.8-423142.3 (m), 139.6,142.2, 144.4 (dt, J = 3.8, 15.9 Hz), 147.7 (dt, J = 4.5, 16.6 Hz), 161.3, 162.4.
MS (ESI): positive mode *mlz =* 503.0 ([M]⁺); MS (ESI): negative mode *mlz =* 171.0 ([C₇H₇SO₃]⁻); ESI HRMS: calcd for C₂₀H₁₂F₄O₃I⁺: 502.9762; found: 502.9769.

### Example 06: Preparation of (4-Methoxyphenyl) [4-(2,3,5,6-tetrafluorophenoxycarbonyl)phenyl]iodonium iodide 22:

(4-Methoxyphenyl)[4-(2,3,5,6-tetrafluorophenoxycarbonyl)phenyl]iodonium tosylate **21** (1.19 g, 1.76 mmol) was dissolved in CH₂Cl₂ (20 mL). After addition of saturated Nal (10 mL), the mixture was vigorously stirred for 15 min and centrifuged (4000 rpm, 15 °C, 10 min). The aqueous solution and precipitate were separated off, saturated Nal (10 mL) was added and the mixture was vigorously stirred for 15 min and centrifuged (x3). The organic fraction was filtered, dried and concentrated under reduced pressure. The residue was recrystallized from CH₂Cl₂/Et₂O, the precipitate was filtered off, washed with acetone (10 mL) and Et₂O (80 mL) to give the title compound (0.29 g, 26%) as an off-white solid. The substance could be stored at 4 °C under argon at least for 4 months. However, it was unstable in solution especially at elevated temperatures (dissolved in DMF or DMSO it was unstable already at ambient temperature).
¹H-NMR(CDCl₃, 300 MHz): δ = 3.79 (s, 3 H), 6.65-6.73 (m, 2 H), 6.95-7.10 (m, 1 H), 7.56 (d, *J* = 8.9 Hz, 2 H), 7.81-8.05 (m, 3 H), 8.21-8.35 (m, 1 H);
¹⁹F-NMR (CDCl₃, 282.4 MHz): δ= -152.63, -138.72;
¹³C-NMR(CDCl₃, 75.5 MHz): δ = 55.5, 82.3, 103.0, 103.5 (t, *J* = 22.6 Hz), 116.4, 126.6, 128.8, 131.9, 138.3, 138.6-142.5 (m), 144.2-144.7 (m), 144.5-144.9 (m), 159.5, 162.2.
MS (ESI): positive mode *mlz =* 502.9 ([M]⁺); MS (ESI): negative mode *mlz =* 126.9 ([I]⁻); ESI HRMS: calcd for C₂₀H₁₂F₄O₃I⁺: 502.9762; found: 502.9741.

### Example 07: Prepartion of (4-Methoxyphenyl) [4-(2,3,5,6-tetrafluorophenoxycarbonyl)phenyl]iodonium perchlorate 4a

To a solution of (4-methoxyphenyl)[4-(2,3,5,6-tetrafluorophenoxycarbonyl)phenyl]-iodonium iodide **22** (0.2 g, 0.32 mmol) in acetone (12 mL) and AgClO₄ (66 mg, 0.32 mmol) was added. The reaction mixture was shaken for 1 min, precipitated Agl was separated by centrifugation. The supernatant was concentrated under reduced pressure and the residue was recrystallized from CH₂Cl₂/Et₂O to give the title compound (168 mg, 88%) as a colorless solid.
¹H-NMR(CDCl₃, 300 MHz): δ = 3.80 (s, 3 H), 6.88-6.98 (m, 2 H), 7.06 (tt, *J* = 9.9, 7.1 Hz, 1 H), 8.04-8.13 (m, 2 H), 8.14-8.18 (m, 2 H), 8.21-8.26 (m, 2 H); ¹⁹F-NMR (CDCl₃, 282.4 MHz): δ= -152.52, -138.48;
¹³C-NMR(CDCl₃, 75.5 MHz): δ = 55.5, 101.1, 103.8 (t, *J* = 21.1 Hz), 118.4, 120.1, 130.6, 133.5, 135.1, 138.3, 138.7-139.3 (m), 142.1-144.4 (m), 147.5-147.7 (m), 161.0, 163.4.
MS (ESI): positive mode *m*/*z* = 503.0 ([M]⁺); ESI HRMS: calcd for C₂₀H₁₂F₄O₃I⁺: 502.9762; found: 502.9769. MS (ESI): positive mode *m*/*z* = 503.0 ([M]⁺); MS (ESI): negative mode *m*/*z* = 171.0 ([C₇H₇SO₃]⁻); ESI HRMS: calcd for C₂₀H₁₂F₄O₃I⁺: 502.9762; found: 502.9749.

### Example 08: Preparation of 2,3,5,6-tetrafluorophenyl 4-fluorobenzoate 24:

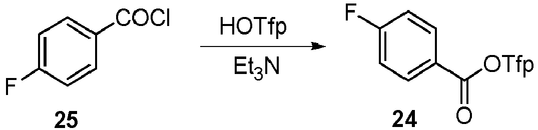

Et₃N (0.54 mL, 0.39 g, 3.87 mmol) was added dropwise to a vigorously stirred solution of 4-fluorobenzoyl chloride **25** (0.46 mL, 0.61 g, 3.87 mmol) and 2,3,5,6-tetrafluorophenol (0.64 g, 3.87 mmol) in Et₂O (30 mL) and the stirring was continued for a further 10 min. The reaction mixture was filtered, washed with H₂O (15 mL) and brine (2×10 mL), dried and concentrated under reduced pressure. The residue was recrystallized from hexane to give **24** (0.41 g) as a colorless solid. The mother liquor was concentrated under reduced pressure and the residue was recrystallized from hexane to give a second crop of **24** (0.45 g, total 77%).
*R*_{f} = 0.62, EtOAc:hexane = 1:10.
¹H-NMR(CDCl₃, 300 MHz): δ = 7.07 (tt, J = 9.9, 7.1 Hz, 1 H), 7.21-7.30 (m, 2 H), 8.21-8.37 (m, 2 H);
¹⁹F-NMR(CDCl₃, 282.4 MHz): δ = -152.75, -138.93, -102.24;
¹³C-NMR(CDCl₃, 75.5 MHz): δ = 103.4 (t, *J* = 21.9 Hz), 116.2 (d, *J* = 21.9 Hz), 123.5 (d, *J* = 1.5 Hz), 133.5 (d, *J* = 2.3 Hz), 139.0-142.7 (m), 144.5-145.1 (m), 147.3-148.2 (m), 163.3 (d, *J* = 259.7 Hz), 168.5. MS (ESI): positive mode *m*/*z =* 288.3 ([M]⁺). MS (El, 70 eV): m/z (%): 165.0 [C₆HF₄O⁺] (10), 123.0 [C₆H₄FO⁺] (100), 95.0 [C₆H₄F⁺] (10).

### Example 09: Preparation of 2,3,5,6-tetrafluorophenyl 4-[¹⁸F]fluorobenzoate 3a

Aqueous [¹⁸F]fluoride (0.05-50 GBq) was trapped on a anion-exchange resin (QMA or Chromafix^{®} 30-PS-HCO₃ cartridge). It should be noted, that in the case of QMA cartridges, the aqueous [¹⁸F]fluoride was loaded onto the cartridge from the male side, whereas MeOH flushing and ¹⁸F⁻elution was done from the female side of the cartridge. If the QMA cartridge has been loaded, flushed and eluted from the female side only, sometimes a significant amount of [¹⁸F]fluoride remained on the resin (this is probably because QMA-light (46 mg) cartridges have a single frit on the male side but four on the female side).The cartridge was washed with MeOH (1 mL) and [¹⁸F]fluoride was eluted into a reaction vial with a solution of (4-methoxyphenyl)[4-(2,3,5,6-tetrafluorophenoxycarbonyl)phenyl]iodonium perchlorate (5 mg) **4a** in MeOH (0.5 mL). Methanol was evaporated under reduced pressure at 70 °C within 2-3 min. After cooling to room temperature DMSO (500 µL) was added. The reaction mixture was stirred at 130 °C for 10 min. Subsequently the mixture was cooled down to room temperature, water (4 mL) was added and the reaction mixture was shaken vigorously for 30 s. Analysis of the mixture by radio-HPLC showed formation of the desired ¹⁸F-labelled active ester in 24% RCY. HPLC conditions: column: Chromolith^{®} SpeedROD RP-18e (Merck, Darmstadt Germany), 50x4.6 mm; eluent: 50% MeCN; flow rate: 3 mL/min.

### Example 10: Preparation of 2,3,5,6-tetrafluorophenyl 6-[¹⁸F]fluoropyridazine-3-carboxylate (3e)

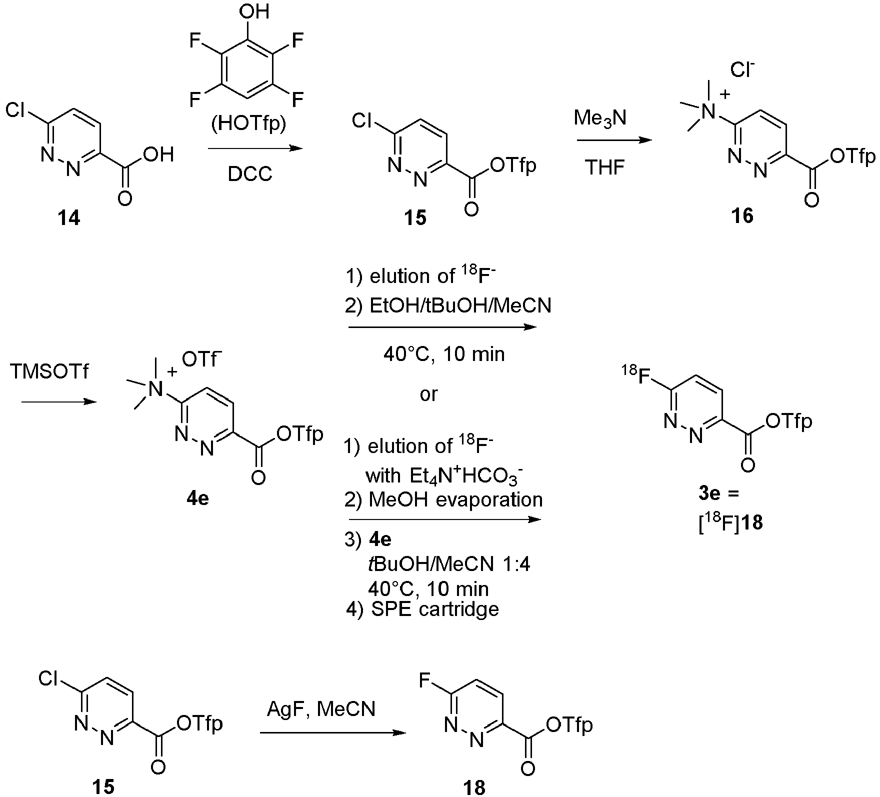

**3e** can be prepared from radiolabelled precursor **4e** using one of two alternative radiolabelling procedures. According to the first one ¹⁸F⁻ is fixed on an anion exchange resin. The resin is washed with EtOH and drained. ¹⁸F⁻ is eluted with an ethanolic solution of **4e.** The resin is additionally washed with tBuOH/MeCN 1:4 and the collected eluates are heated for a short time. Finally **3e** is isolated via SPE. Alternatively **3e** is eluted with methanolic tetraethylammonium bicarbonate, the solvent is evaporated and the residue is taken up in a solution of **4e** in tBuOH/MeCN 1:4 and heated for a short time. Finally the desired active ester is isolated via SPE.

Radiolabelling precursor **4e** can be prepared from commercially available precursor **14** as follows:
Esterification with 1,2,5,6-tetrafluorphenol/DCC, treatment of the intermediate active ester **15** with anhydrous trimethylamine in THF followed by anion metathesis using TMSOTf. The reference compound 18 can be prepared from intermediate 15 by treatment with AgF under anhydrous conditions.

### Example 11: Preparation 2,3,5,6-tetrafluorophenyl 5-[¹⁸F]fluoropyrazine-2-carboxylate (3f)

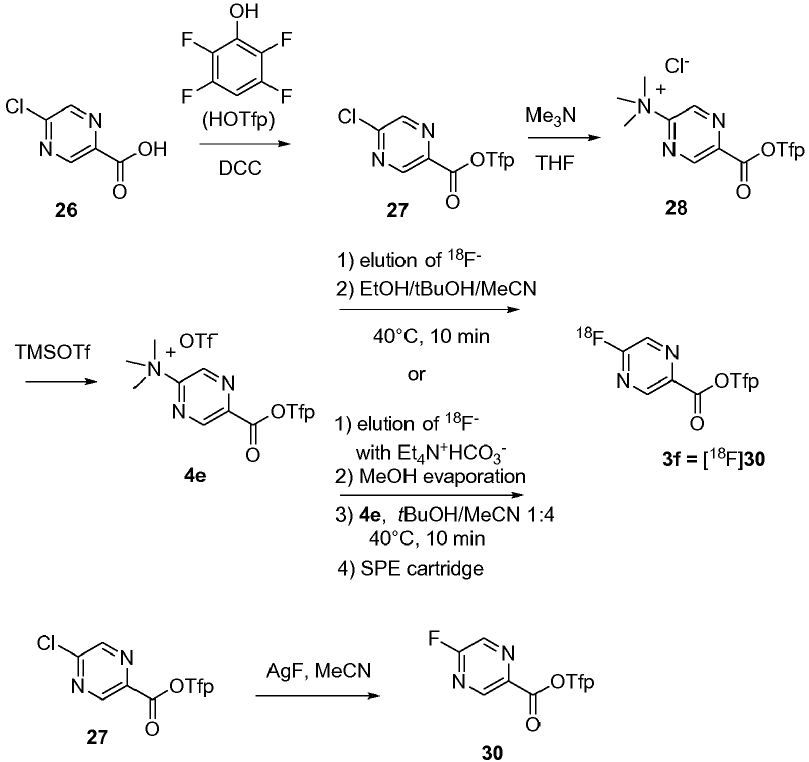

Compound **3f** can be prepared exactly as **3e** as decribed in the Example 10.

### Example 12: Preparation of 2,3,5,6-tetrafluorophenyl 4-[¹⁸F]fluoro-2,3,5,6-tetrafluorobenzoate (3d)

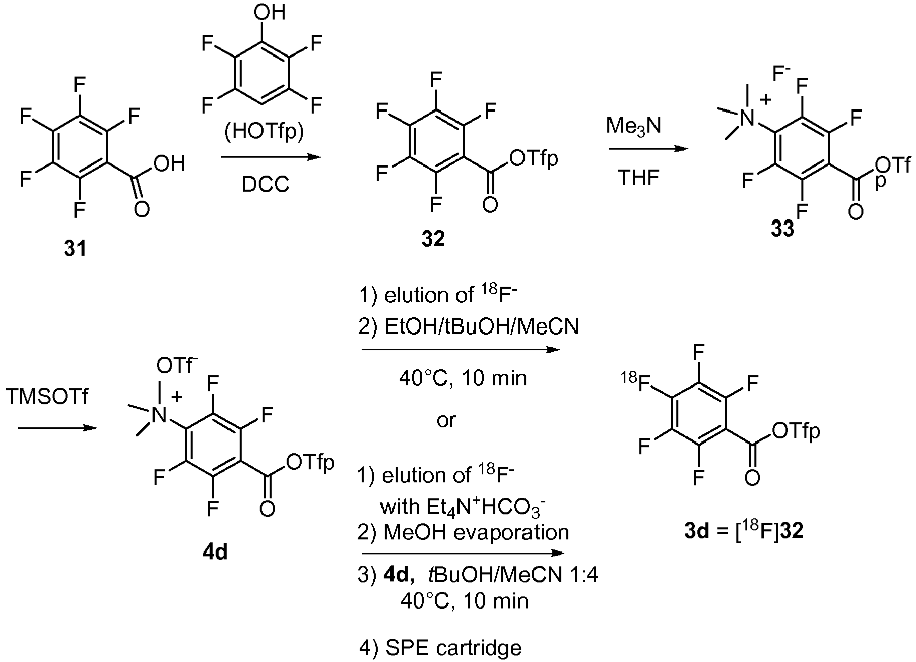

Compound 3d can be prepared exactly as 3e as decribed in the Example 10.

### Example 13: Preparation of (2S)-2-({[(1S)-1-carboxy-5-{[6-[¹⁸F]fluoropyridazin-3-yl]formamido}pentyl]carbamoyl}amino)butanedioic acid (1-17)

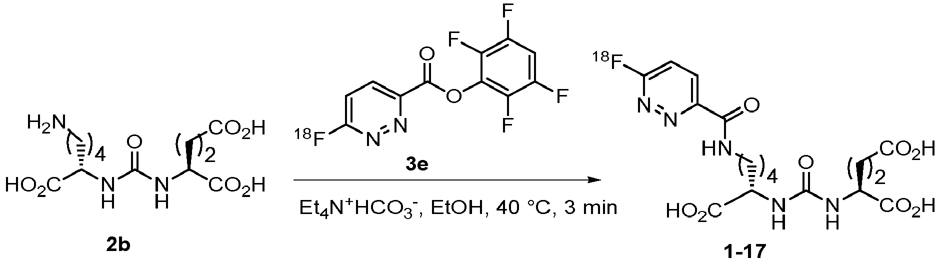

Compound 1-17 can be prepared exactly as [¹⁸F]DCFPyL (1-10) as decribed in the Example 2.

### Example 14: Preparation of (2S)-2-({[(1S)-1-carboxy-5-{[5-[¹⁸F]fluoropyrazin-2-yl]formamido}pentyl]carbamoyl)amino)butanedioic acid (1-29)

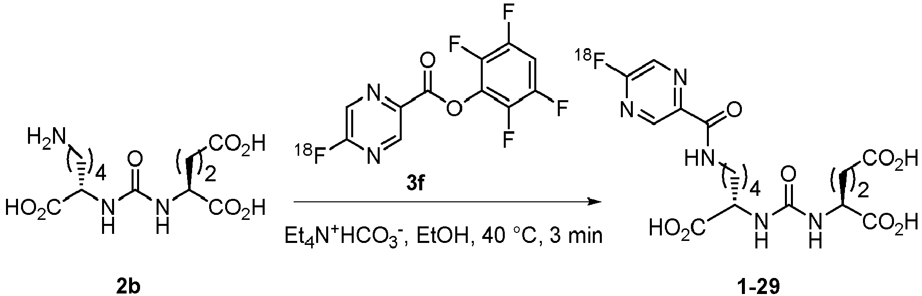

Compound 1-29 can be prepared exactly as [¹⁸F]DCFPyL ([¹⁸F] 1-10) as decribed in the Example 2.

### Example 15: Preparation of (2S)-2-({[(1S)-1-carboxy-5-(3-{[6-[¹⁸F]fluoropyridin-3-yl]formamido}propanamido)pentyl]carbamoyl}amino)-butanedioic acid (1-31)

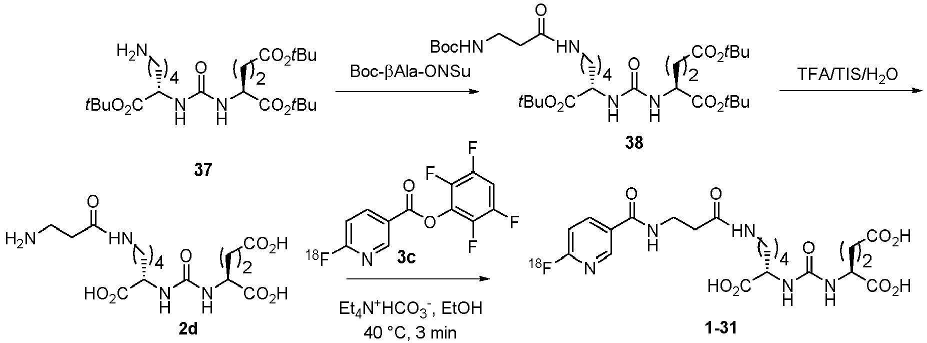

Compound 1-33 can be prepared analogously to [¹⁸F]DCFPyL ([¹⁸F]**1-10**) using 2d instead of 2b. 2d can in turn be prepared using conventional methods of peptide synthesis, for example, via acylation of known 37 with Boc-βAla-ONSu followed by cleavage of protecting groups.

### Example 16: Preparation of (2S)-2-({[(1S)-1-carboxy-5-(2-{[6-[¹⁸F]fluoropyridazin-3-yl]formamido}acetamido)pentyl]carbamoyl}amino)-butanedioic acid (1-32)

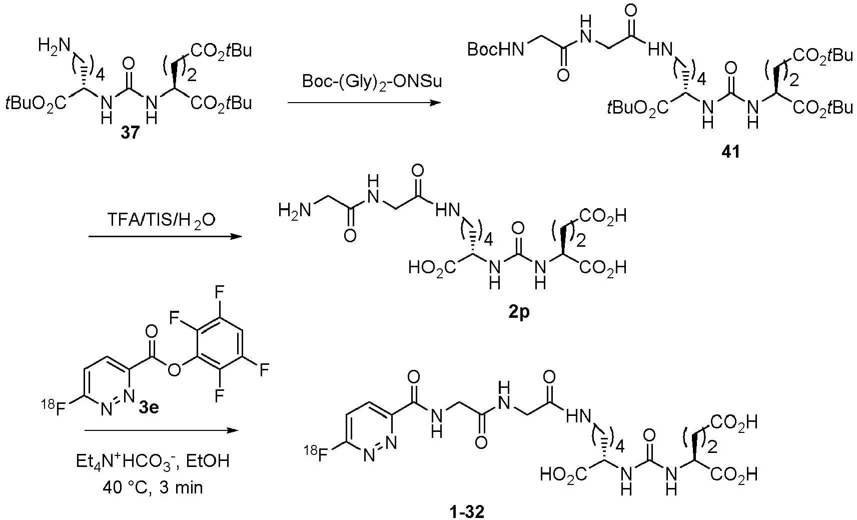

Compound **1-32** can be prepared analogously to [¹⁸F]DCFPyL (**1-10**) using **2p** instead of **2b. 2p** can in turn be prepared using conventional methods of peptide synthesis, for example, via acylation of known **37** with Boc-Gly-Gly-ONSu followed by cleavage of protecting groups.

### Example 17: Preparation of (2S)-2-({[(1S)-1-carboxy-5-[(4S)-4-carboxy-4-{[4-[¹⁸F]fluoro-2,3,5,6-tetrafluorophenyl]formamido}butanamido]pentyl]carba-moyl}amino)butanedioic acid (1-33)

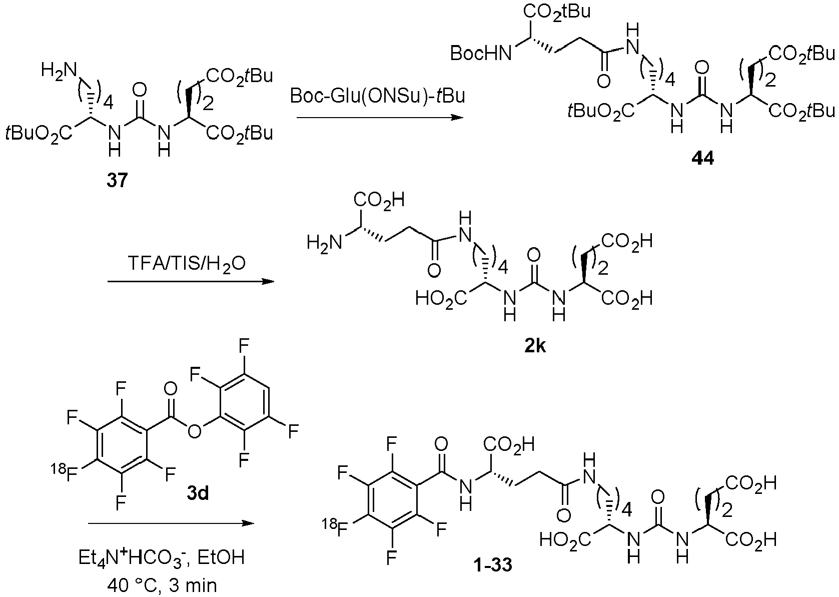

Compound **1-33** can be prepared analogously to [¹⁸F]DCFPyL (**1-10**) using **2k** instead of **2b. 1-33** can in turn be prepared using conventional methods of peptide synthesis, for example, via acylation of known **37** with Boc-Glu(ONSu)-O*t*Bu followed by cleavage of protecting groups.

### Example 18: Preparation of (2S)-2-({[(1S)-1-carboxy-5-[(4S)-4-carboxy-4-[(4S)-4-carboxy-4-{[6-[¹⁸F]fluoropyridin-3-yl]formamido}butanamido]butanamido]-pentyl]carbamoyl}amino)butanedioic acid(1-34)

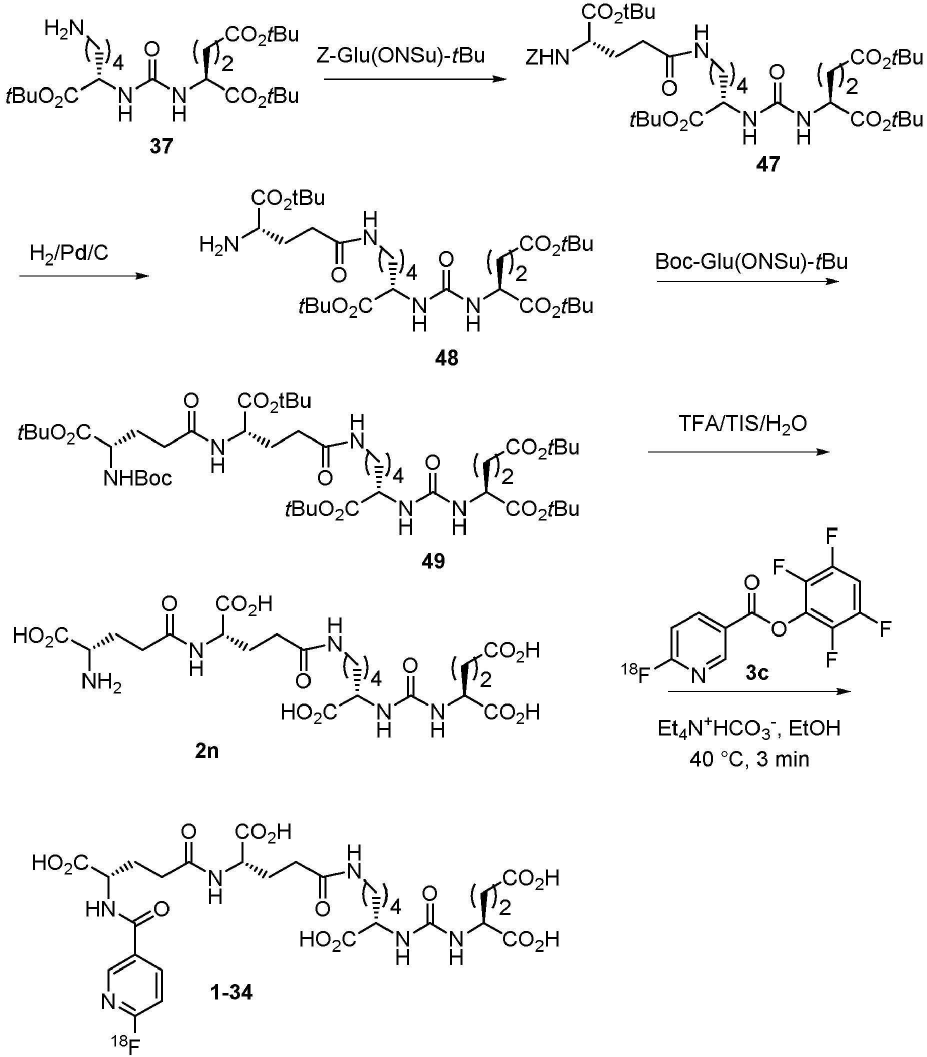

Compound 1-34 can be prepared analogously to [¹⁸F]DCFPyL ([¹⁸F]**1-10**) using 2n instead of 2b. 2n can in turn be prepared using conventional methods of peptide synthesis, for example, via acylation of known **37** with Z-Glu(ONSu)-O*t*Bu followed by cleavage of Z (Z = Boc = tert-butyloxycarbonyl) group, followed by acylation of intermediate 49 with Boc-Glu(ONSu)-O*t*Bu and final deprotection with TFA.

### Example 19: Comparison of [¹⁸F]DCFPyL ([¹⁸F]1-10) with [⁶⁸Ga]HBED-CC (50) in PSMA⁺-PCa xenograft mice

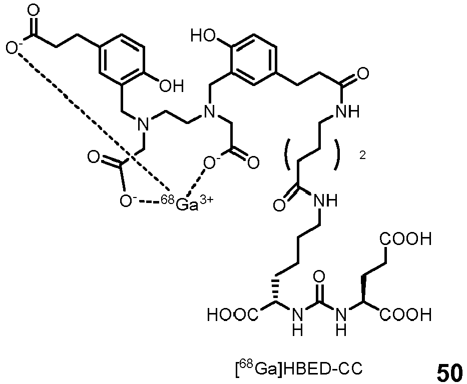

### Methods:

Five 8-week-old male C.B-Igh-1b/IcrTac-Prkdcscid mice (Taconic) were injected subcutaneously in the neck region with 4×10⁶ LNCaP-C4-2 prostate tumor cells. Three weeks after tumor implantation, two PET measurements with [¹⁸F]DCFPyL ([¹⁸F]**1-10**) and [⁶⁸Ga]HBED-CC **(50),** respectively, were performed using an Inveon µPET/CT scanner (Siemens) with a resolution of 1.4 mm FWHM at center of field of view. The first PET measurement was immediately followed by a CT scan during which the animal was left in the same position.

Both tracers were injected i.v. through the lateral tail vein ([¹⁸F]**1-10**: 21-32 MBq; [⁶⁸Ga]HBED-CC: 19-33 MBq, on two consecutive days). Emission data collection started 60 min after injection and lasted 45 min. Image reconstruction was performed using Fourier rebinning and an ordered subset expectation maximization (3D-OSEM) procedure, yielding images with voxel sizes of 0.78 x 0.78 x 0.80 mm.

Image analysis was performed with VINCI 4.0 (MPI for Metabolism Research, Cologne, Germany). Tracer uptake in the tumor as well as liver, kidney, and background was determined using volumes of interest (VOIs) covering the respective structure. Significant differences were assessed using a two-way repeated measures ANOVA with factors "structure" and "tracer", followed by Holm-Sidak post hoc comparison.

Tumor sizes were measured by drawing accurate tumor VOIs in the CT images, and extracting VOI volume. Tracer uptake was correlated tumor size using the Pearson correlation test.

### Results

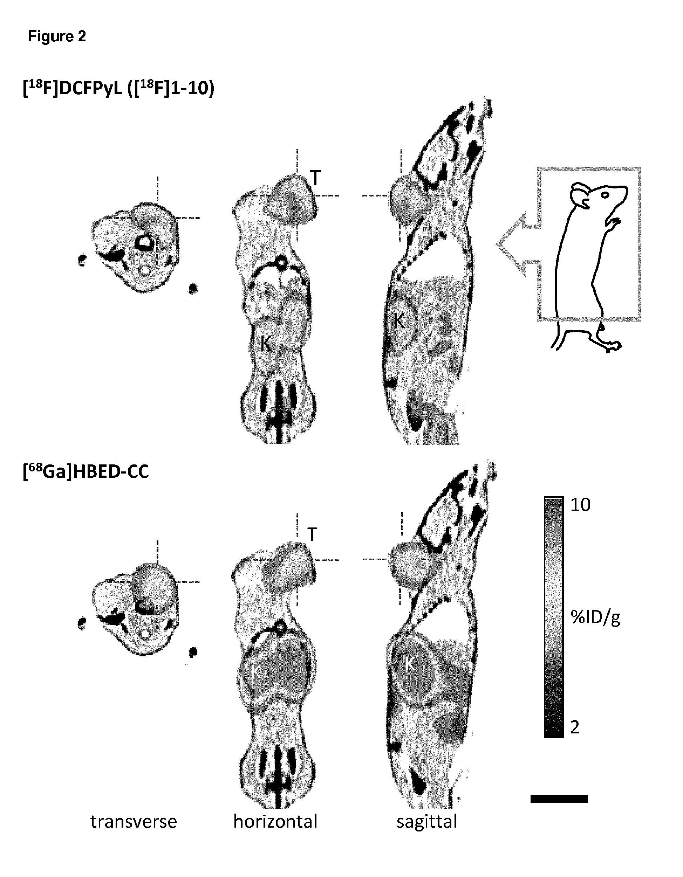

As shown in figure 2, tumor tracer uptake was similar for the two tracers (Figure 2): 1.4 ± 1.7 %ID/g for [¹⁸F]**1-10**, and 1.8 ± 2.0 %ID/g for [⁶⁸Ga]HBED-CC **(50)** (not significant). There was a significant positive correlation between tumor size and tracer uptake for both tracers: R=0.97, p=0.0059 for [¹⁸F]**1-10**, and R=0.98, p=0.0019 for [⁶⁸Ga]HBED-CC **(50).** Signal-to-noise ratio (tumor versus background) was nearly identical as well with 5.9 ± 4.2 for [¹⁸F]**1-10**, and 6.7 ± 6.9 for [⁶⁸Ga]HBED-CC **(50).**

While liver uptake was similar as well (1.1 ± 0.5 %ID/g for [¹⁸F]**1-10**, and 0.7 ± 0.2 %ID/g for [⁶⁸Ga]HBED-CC **(50);** not significant), [¹⁸F]**1-10** uptake in the kidney (3.0 ± 1.9 %ID/g) was significantly lower than [⁶⁸Ga]HBED-CC **(50)** uptake (13.9 ± 6.4 %ID/g; F(3,12)=25.6, p<0.0001 for factor interaction, post-hoc p<0.05).

## Claims

1. A method for preparing a compound of formula (I) 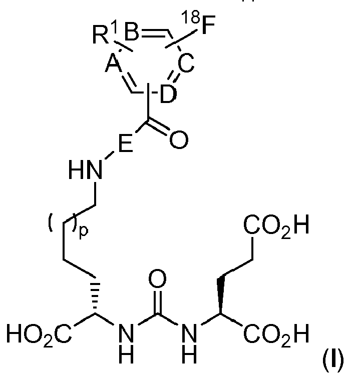 wherein
**A, B, C,** and D represent independently of each other C-H, C-F or N; and not more than two of **A,** B, C, and D represent N;
E represents a covalent bond or 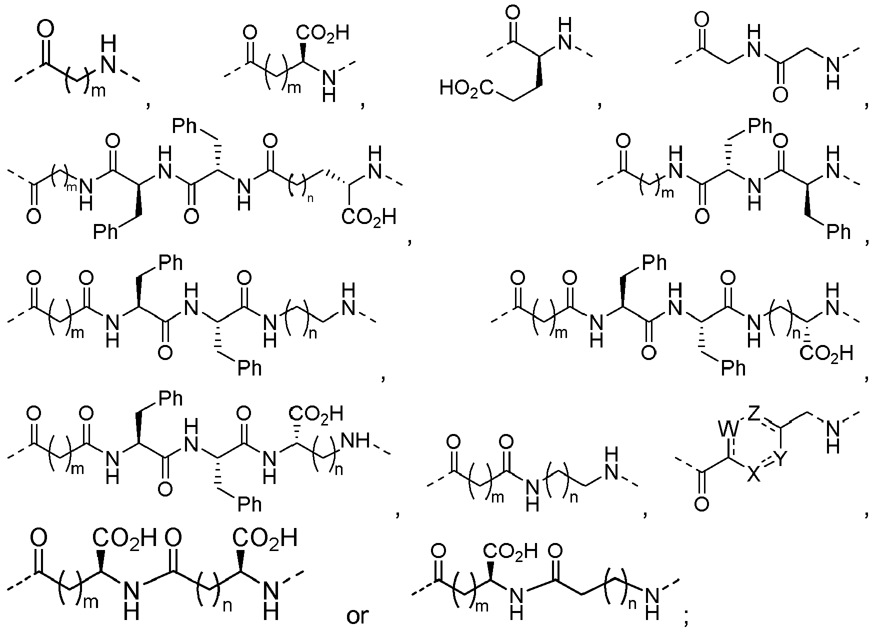
**R¹** represents C₁-C₃ alkyl, C₁-C₃ alkoxy, C₁-C₃ haloalkyl, C₁-C₃ haloalkoxy, C₂-C₄ alkylcarbonyl, C₂-C₄ alkyloxycarbonyl, C₂-C₄ alkylcarboxy, aryloxy, arylcarboxy, cyano, or nitro;
**n** is an integer selected from 0 to 10;
**m** is an integer selected from 1 to 18;
**p** is an integer selected from 0 to 10;
**X, Y, W,** and **Z** represent independently of each other -CH₂-, -CH-, -NH-or -N-;
-̅-̅-̅-̅ represents a single or double bond;
and diastereomers, entantiomers, hydrates, and salts thereof;
comprising the steps:
(A) providing a solution of a compound of formulae (II) in a polar protic solvent or in a solvent mixture containing a polar protic solvent containing at least one base 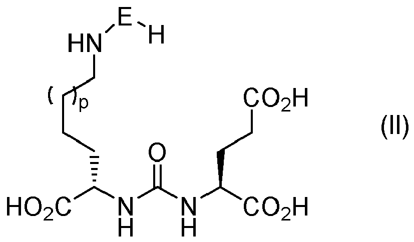 wherein E and p have the meanings as defined above,
(B) providing a solution of a compound of formula (III) 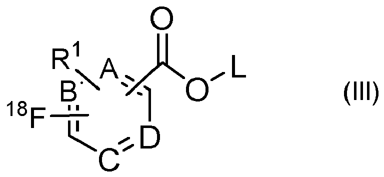 wherein
**A, B, C, D** and **R¹** have the meanings as defined above, and
OL represents a leaving group
in a polar protic solvent or in a solvent mixture containing a polar protic solvent;
(C) mixing the solution of the compound of formula (II) and the solution of the compound of formula (III) and allowing the compound of formula (II) to react with the compound of formula (III) in order to obtain the compound of formula (I),
(D) purifying the compound of formula (I) preferably by using isotonic sodium chloride solution.

2. Method according to claim 1, wherein step (C) is performed at a reaction temperature T2 which is in the range of 30°C to 60°C and during a reaction time t2 which is 1-30 min and at a pH value of the reaction solution which is in the range of 7.0-11.0.

3. Method according to claim 1 or 2, wherein **L** in the leaving group **OL** represents: 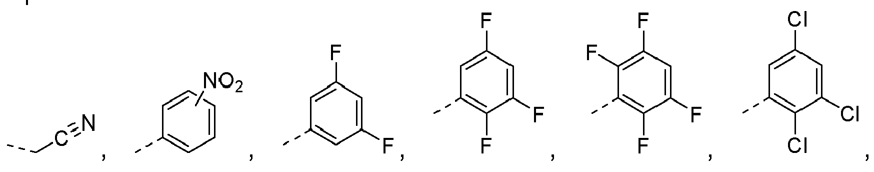 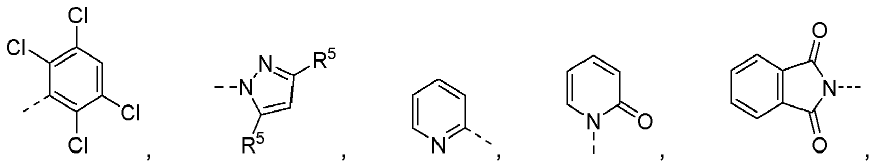 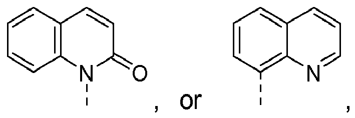 and wherein R⁵ is selected from methyl, ethyl, or n-propyl;

4. Method according to claim 1, 2 or 3 further comprising the following step (E) after the step (D):
(E) sterilizing the solution of the compound of formula (I) via steril filtration.

5. Method according to claim 1, 2, 3 or 4 further comprising the following steps (A1) - (A8) after step (A) and before step (B):
(A1) providing a solution of a compound of the formula (IV) in at least one polar protic solvent or in a solvent mixture containing a polar protic solvent,
wherein optionally the solution further contains a salt; 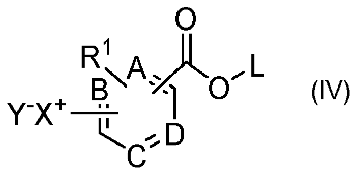 wherein
**A, B, C, D, OL** and **R¹** have the same meanings as defined in claim 1;
**X** represents **NR²**₃**, IR³, SR³₂;**
**Y** represents Br, I, BF₄, O₂CCF₃, OSO₂CF₃, ClO₄, NO₂, OSO₂C₆H₄CH₃, OSO₂CH₃
**R²** represents C₁ - C₄ alkyl; and
**R³** represents aryl;
(A2) providing an aqueous solution of [¹⁸F]fluoride;
(A3) loading the aqueous solution of [¹⁸F]fluoride onto an anion exchange resin;
(A4) washing the anion exchange resin with a polar protic solvent or with a polar aprotic solvent;
(A5) flushing of the solvent with air or inert gas flow;
(A6) eluting of [¹⁸F]fluoride with the solution of the compound of formula (IV) provided in step (A1) and diluting of the resulting solution with an aprotic solvent or with at least one C₃ - C₆ alcohol or with a solvent mixture of at least one C₃ - C₆ alcohol and an aprotic solvent;
or
eluting of [¹⁸F]fluoride with the solution of the compound of formula (IV) provided in step (A1), concentrating of the resulting solution and redissolving of the residue in an aprotic solvent;
(A7) allowing the compound of formula (IV) to react with [¹⁸F]fluoride in order to obtain the compound of the formula (III); 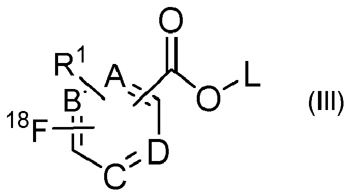 wherein **A, B, C, D, OL** and **R¹** have the same meanings as defined in claim 1; and
(A8) purifying of the compound of formula (III).

6. Method according to any one of the claims 1 - 5 further comprising the following step (F) after the step (D) or (E):
(F) preparing a pharmaceutical composition containing the solution of the compound of formula (I).

7. Method according to any one of the claims 1 - 6, wherein the base in step (A) is an organic nitrogen-containing base or a bicarbonate.

8. Method according to claim 7, wherein the organic nitrogen-containing base or the bicarbonate is selected from the group consisting of:
LiHCO₃, NaHCO₃, KHCO₃, CsHCO₃, Me₄NHCO₃, Et₄NHCO₃, *n*-Pr₄NHCO₃, *i*-Pr₄NHCO₃, *n*-Bu₄NHCO₃, *i*-Bu₄NHCO₃, Et₃N, pyridine, lutidine, collidine, diisopropylethylamine, *n*-Pr₃N, *i*-Pr₃N, *n*-Bu₃N, *i*-Bu₃N, Oct₃N, N-methyl-morpholine, N-ethylmorpholine, N-methylpiperidine, N-ethylpiperidine, *N,N*-dicyclohexylmethylamine, *N,N*-dimethylcyclohexylamine, N-methyldibutylamine, *N*-ethyldicyclohexylamine, *N,N*-dimethylbutylamine, and *N,N*-dimethylhexylamine

9. Method according to any one of the claims 1 - 8, wherein the polar protic solvent is an anhydrous polar protic solvent, especially anhydrous MeOH or anhydrous EtOH or a mixture thereof.

10. Method according to any one of the claims 1 - 9, wherein the compound (III) is

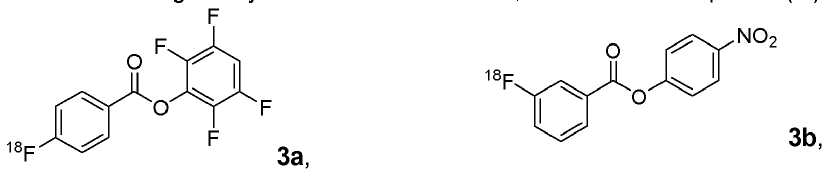

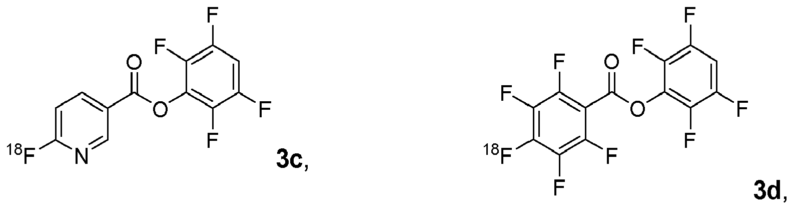

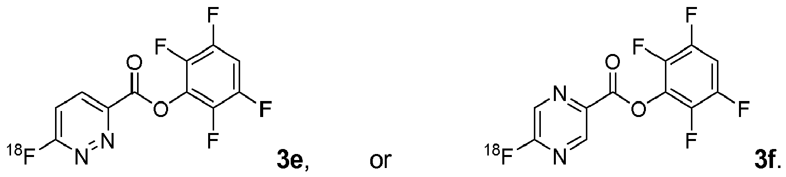

11. Method according to any one of the claims 5 - 10, wherein the [¹⁸F]fluoride is trapped on an anion exchange resin and then eluted directly.

12. Method according to any one of the claims 5 - 11, wherein the C₃ - C₆ alcohol is *t*BuOH.

13. Method according to any one of the claims 1 - 12, wherein the compound of the formula (I) is selected from the group consisting of compounds **1-3, 1-10, 1-14, 1-17, 1-29, 1-31, 1-32, 1-33,** and **1-34:** 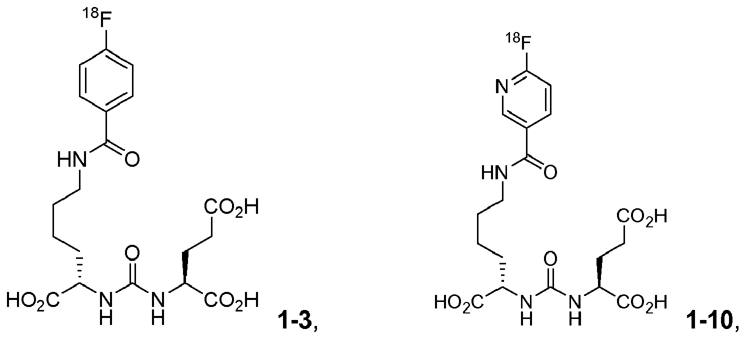 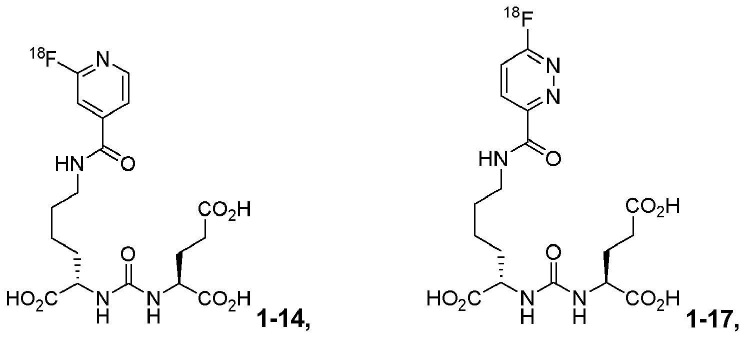 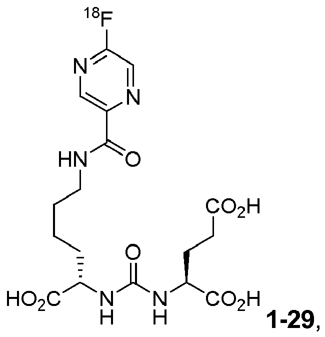 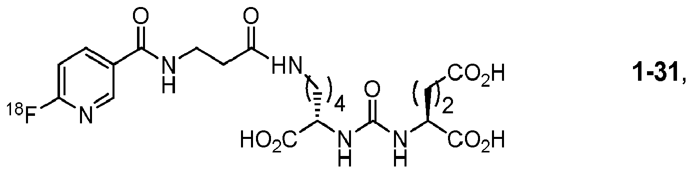 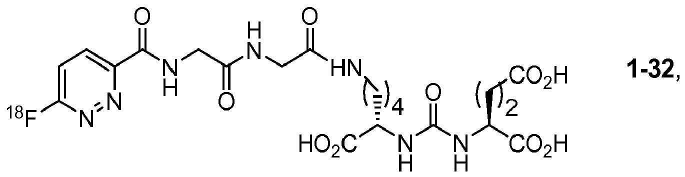 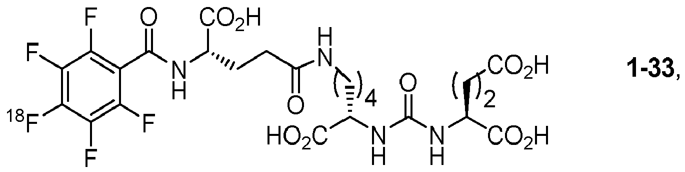 and

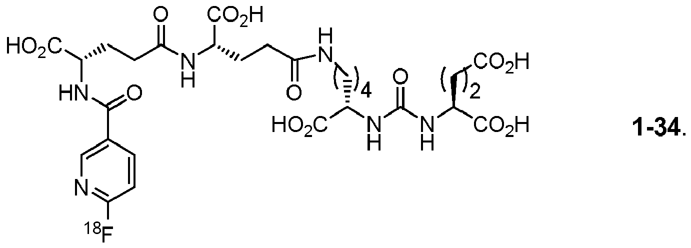

14. Pharmaceutical composition containing at least one compound of formula (I) as defined in claim 1 together with at least one pharmaceutically acceptable solvent, ingredient and/or diluent.

15. Pharmaceutical composition according to claim 14 for use in imaging prostate cancer cells or prostate cancerous tissue.
